(19) Europäisches Patentamt
European Patent Office
Office européen des brevets

(11) EP 0 874 811 B1

(12) **EUROPÄISCHE PATENTSCHRIFT**

(45) Veröffentlichungstag und Bekanntmachung des Hinweises auf die Patenterteilung:
**13.06.2001 Patentblatt 2001/24**

(21) Anmeldenummer: **96943038.8**

(22) Anmeldetag: **05.12.1996**

(51) Int Cl.[7]: **C07C 319/28**, C07C 319/20, C07C 323/52

(86) Internationale Anmeldenummer:
**PCT/EP96/05437**

(87) Internationale Veröffentlichungsnummer:
**WO 97/23452 (03.07.1997 Gazette 1997/29)**

(54) **VERFAHREN ZUR GEWINNUNG VON 2-HYDROXY-4-METHYLTHIOBUTTERSÄURE (MHA)**
PROCESS FOR OBTAINING 2-HYDROXY-4-METHYLTHIOBUTYRIC ACID (MHA)
PROCEDE D'OBTENTION D'ACIDE 2-HYDROXY-4-METHYLTHIOBUTRIQUE (MHA)

(84) Benannte Vertragsstaaten:
**BE DE ES FR GB IT NL**

(30) Priorität: **23.12.1995 DE 19548538**

(43) Veröffentlichungstag der Anmeldung:
**04.11.1998 Patentblatt 1998/45**

(73) Patentinhaber: **Degussa AG**
**40474 Düsseldorf (DE)**

(72) Erfinder:
- **HASSEBERG, Hans-Albrecht**
  **D-63584 Gründau (DE)**
- **HASSELBACH, Hans-Joachim**
  **D-63571 Gelnhausen (DE)**
- **HUTHMACHER, Klaus**
  **D-63571 Gelnhausen (DE)**
- **HÄFNER, Volker**
  **D-63505 Langenselbold (DE)**
- **HEINZEL, Harald**
  **D-60488 Frankfurt (DE)**
- **JÄGER, Barbara**
  **D-63579 Freigericht (DE)**

(56) Entgegenhaltungen:
**EP-A- 0 142 488**
**EP-A- 0 143 100**
**EP-A- 0 330 527**
**WO-A-94/28717**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 874 811 B1

## Beschreibung

Die Erfindung betrifft ein Verfahren zur Gewinnung von 2-Hydroxy-4-methylthiobuttersäure (MHA), bei dem das MHA aus einem Reaktionsgemisch isoliert wird, welches durch Anlagerung von Blausäure (HCN) an Methylmercaptopropionaldehyd (MMP) und Hydrolyse des dabei erhaltenen Methylmercaptopropionaldehyd-Cyanhydrins (MMP-CH) mit Schwefelsäure erhalten wird, wobei das Reaktionsgemisch mit einem im wesentlichen mit Wasser nicht mischbaren organischen Lösungsmittel in einem Flüssig/Flüssig-Extraktionssystem in Kontakt gebracht wird, um eine Extraktionslösung zu bilden, die das Lösungsmittel und aus dem Reaktionsgemisch überführtes MHA aufweist, und das MHA als Extrakt aus dieser Extraktionslösung durch Eindampfen gewonnen wird.

2-Hydroxy-4-methylthiobuttersäure (MHA) ist das Hydroxyanaloge der essentiellen Aminosäure Methionin in racemischer Form und ist wie diese ein wichtiger Zusatzstoff in der Tierernährung. In der Geflügelaufzucht zeigt MHA ähnliche wachstumsstimulierende Eigenschaften wie die dafür bekannte Aminosäure. Aber auch in anderen Bereichen der Tierernährung findet das Additiv zunehmendes Interesse.

Eingesetzt wird MHA meist in Form wässriger Konzentrate, wobei diese neben dem Monomeren noch einen gewissen Anteil an Oligomeren, hauptsächlich die di- und trimeren linearen Estersäuren enthalten. Der Gehalt an diesen Oligomeren hängt von den Herstellungsbedingungen und der gewählten Konzentration ab. Wegen ihres geringeren nutritiven Wirkungsgrades und des ungünstigen Einflusses auf die Fließeigenschaften infolge Viskositätserhöhung ist es jedoch wünschenswert, ihren prozentualen Anteil möglichst niedrig zu halten. Handelsübliche Formulierungen weisen bei einer Gesamtkonzentration von 88 - 90 Gew.-% bis zu 24 Gew.-%, entsprechend ca. 27 Mol-%, in der Summe an Oligomeren auf, entsprechend einem Monomeren/Oligomeren-Verhältnis von ~ 3 : 1.

Das allgemeine Verfahren zur Herstellung von MHA geht von 3-Methylthiopropionaldehyd, auch als Methylmercaptopropionaldehyd oder MMP bezeichnet, aus, der mit Cyanwasserstoff zum 2-Hydroxy-4-methylthiobutyronitril, auch als MMP-Cyanhydrin oder MMP-CH bezeichnet, umgesetzt wird (Gleichung I).

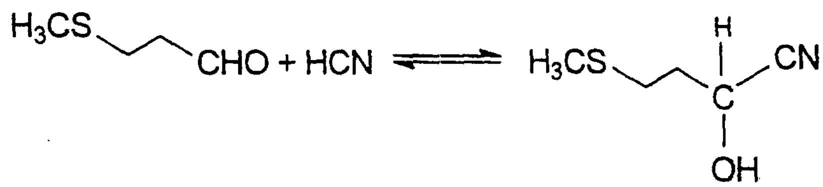

(I)

Das entstandene MMP-Cyanhydrin wird anschließend üblicherweise mit starken Mineralsäuren wie Schwefelsäure oder Salzsäure über die Zwischenstufe des 2-Hydroxy-4-methylthiobutyramids, auch als MHA-Amid bezeichnet (Gleichung II),

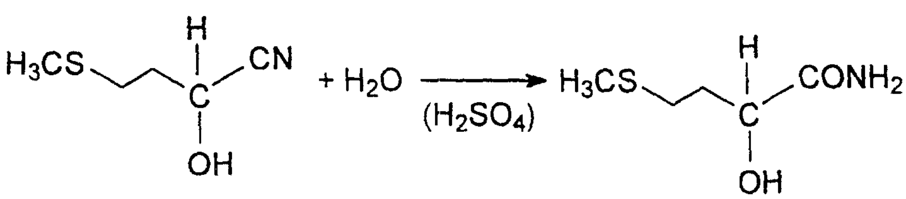


(II)

zum Methioninhydroxyanalogen (MHA) hydrolysiert (Gleichung III).

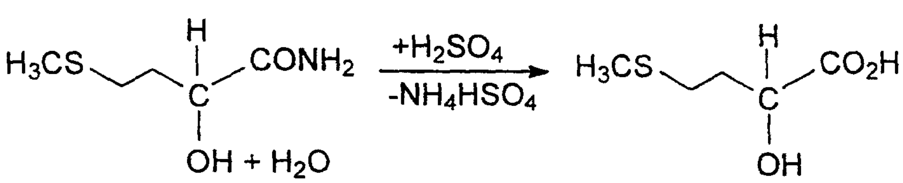


(III)

Diese Hydrolyse kann sowohl ein- als auch zweistufig durchgeführt werden, wobei unter "Stufen" zu verstehen ist, daß zur Hydrolyse des MMP-CH's entweder einmal oder zweimal Mineralsäure und/oder Wasser zugesetzt wird,

d. h. die Anzahl der Stufen entspricht der Anzahl der Zugabevorgänge.

Zum näheren Stand der Technik werden die Druckschriften:

EP-A-0 142 488 = D1,
EP-A-0 143 100 = D2,
EP-A-0 330 527 = D3 und
WO-A-94/28717 ≙ EP 93 924 374 = D4

genannt.

Ein gattungsgemäßes Verfahren zur Gewinnung von MHA ist beispielsweise aus der D1 bekannt. Die D1 bedient sich zur Gewinnung von MHA in flüssiger Form als hochkonzentrierte wässrige Lösung der Zweistufenhydrolyse mit Schwefelsäure.

Gemäß der D1 erhält man MHA nach der, unter definierten Konzentrations- und Temperaturbedingungen über die Amidstufe mit überschüssiger Mineralsäure durchgeführten Hydrolysereaktion, mit Hilfe einer Lösungsmittelextraktion, wobei bestimmte, mit Wasser partiell mischbare Lösungsmittel zur Anwendung kommen.

Entsprechend den Angaben in der D1 ist das kennzeichnende Merkmal des dort beschriebenen Verfahrens in der Gewinnung des MHA's aus der Extraktionslösung zu sehen, welche so 5 durchgeführt wird, daß die Gewinnung das Entfernen des organischen Lösungsmittels in Anwesenheit von zumindest etwa 5 Gew.-% Wasser, bezogen auf den verbleibenden Extrakt (MHA), umfaßt. MHA wird aus der Extraktionslösung durch Destillation gewonnen (siehe Beispiele), wobei die Dampfdestillation bevorzugt ist. Durch Entfernung des Lösungsmittels aus der Extraktionslösung während der Dampfdestillation ist der erhaltene Ablauf eine Mischung aus MHA und Wasser. Die Dampfdestillation wird also demzufolge so geführt, daß der Ablauf mindestens 5 Gew.-% Wasser enthält.

An anderer Stelle wird in der D1 ausgeführt, daß die Kolonnenbedingungen bei der Destillation so reguliert werden, daß überall in der Kolonne, zumindest jedoch in der Bodenfraktion, die flüssige Phase 5 Gew.-% Wasser aufweist.

Daraus folgt, daß ohne Gegenwart einer ausreichenden Menge Wasser während der Gewinnung des MHA aus der Extraktionslösung, die zunehmende Bildung gegebenenfalls unerwünschter Nebenprodukte (Dimere und Oligomere) zu befürchten ist.

Weiterhin dient der Wasserdampf in der Destillation als treibendes Agens zur restlosen Entfernung des Extraktionsmittels aus der MHA-Lösung, z. B. durch Bildung eines tiefsiedenden Azeotropgemisches mit dem entsprechenden Extraktionsmittel.

Weitere Ausführungsformen eines im wesentlichen gattungsgleichen Verfahrens werden in der D2 beschrieben. Im Unterschied zum Oberbegriff der D1 wird die Hydrolyse des MMP-CH's mit einer Mineralsäure geschildert, womit auf die alternative Einsatzmöglichkeit von HCl anstelle von $H_2SO_4$ bezug genommen wird. Es werden insgesamt drei weitere Varianten offenbart, die sich im wesentlichen mit Abwandlungen der Isolierung des MHA's aus dem Mineralsäure-Hydrolysat oder der Aufarbeitung des Extraktes bei der Flüssig/Flüssig-Extraktion befassen.

In einem ersten Aspekt gemäß D2 wird das Hydrolysat ohne vorherige Abtrennung irgendwelcher wesentlicher Fraktionen von darin enthaltenen Feststoffen mit dem organischen Lösungsmittel in Berührung gebracht. Außerdem werden gemäß D2 dabei die Bedingungen der Extraktion so reguliert, daß der Extrakt und das wäßrige Raffinat die einzigen flüssigen Phasen sind, die bei Phasentrennung nach der Extraktion gebildet werden.

Nachteilig bei diesem ersten Aspekt ist, daß die Extraktion mit dem vollen bei der Hydrolyse gebildeten Salzanteil im Hydrolysat belastet wird, was zu einem relativ hohen Massenstrom an Hydrolysat und entsprechend auch an Lösungsmittel führt. Dies hat entsprechend hohe Energiekosten für Lösungsmitteleindampfung und Kondensation sowie Kosten für entsprechenden Lösungsmittelverlust sowie für entsprechend große Aggregate in Extraktion und Eindampfung zur Folge. Eine Verringerung des Betriebs- und Investitionskostenanteils an dieser Stelle des Verfahrens wäre somit wünschenswert (insbesondere im Hinblick auf die Größenordnung einer solchen Anlage und die damit verbundenen Einsparpotentiale).

Das als homogene Flüssigphase aus der Extraktion erhaltene Raffinat muß nach D1 bzw. D2 bzw. D4 durch Strippung oder Destillation von Lösungsmittelresten befreit werden, was einen unerwünschten Zusatzaufwand darstellt.

In einem zweiten Aspekt bezieht sich die D2 auf die Abtrennung des organischen Lösungsmittels aus dem Extrakt. Hierzu wird ausgeführt, daß die Abtrennung bewirkt wird, indem der Extrakt einer Dampfdestillation unterworfen wird, wobei das Lösungsmittel ausgetrieben wird und sich eine Bodenfraktion aus wäßrigem MHA bildet.

Nachteilig bei der Verwendung von Dampf ist insbesondere der vermehrte Anfall des wäßrigen mit Lösungsmittel belasteten Dampfbrüdenkondensats, welches anschließend durch unerwünschten Zusatzaufwand wie Destillation, Strippung von Lösungsmittel befreit werden muß, um es dann an geeigneter Stelle in den Prozeß zurückzuführen oder welches kostenintensiv entsorgt z. B. verbrannt werden muß. Eine Vermeidung von zusätzlichem Strippdampf wäre daher wünschenswert.

Schließlich legt die D2 in einem dritten Aspekt einen Schwerpunkt auf die Art des zur Flüssig/Flüssig-Extraktion einzusetzenden Lösungsmittels. Die zur Wahl eines geeigneten Lösungsmittels zu beachtenden Kriterien umfassen insbesondere folgende Punkte:

- Der Siedepunkt des Lösungsmittels soll zwischen 60° und 200° C liegen;
- der Verteilungskoeffizient für MHA im Gleichgewicht zwischen Hydrolysat und Lösungsmittel soll zumindest etwa zwei betragen;
- der Verteilungskoeffizient des Lösungsmittels im Gleichgewicht zwischen Extrakt und wässriger Phase soll zumindest etwa eins betragen;
- die Löslichkeit von Wasser im Lösungsmittel soll bei Raumtemperatur nicht mehr als etwa 12 Gew.-% betragen.

Der relativ hohe Siedepunktbereich der hier zu verwendenden Lösungmittel von 60 - 200 °C erfordert bei der Eindampfung des Extraktes relativ hohe Temperaturen, die das Produkt schädigen können, sowie zusätzliche Hilfsstoffe wie Strippdampf, was unerwünscht ist.

Einer der wesentlichen Nachteile bei den Verfahren der D1 und D2 besteht jedoch in der großen Salzfracht, welche bei der Verseifung entsteht und die das Verfahren zur Isolierung des MHA's in einem ansonsten relativ eleganten Flüssig/Flüssig-Extraktionsverfahren doch noch unvorteilhaft belastet. Eine Aufarbeitung des zwangsweise anfallenden Ammoniumsalzgemisches ist meist nicht rentabel, das Deponieren ist aus ökologischen Gesichtspunkten sehr bedenklich und dürfte selbst an Standorten mit weniger strengen Auflagen in absehbarer Zeit gesetzlich untersagt werden.

An Versuchen, die Salzabfallfracht aus der Verseifung zu reduzieren oder gar zu vermeiden, hat es zwar nicht gefehlt, die dabei erzielten Vorteile wurden jedoch in aller Regel unter Inkaufnahme einer Reihe anderer Nachteile oder Verzicht auf das elegante Handling gemäß D1 und D2 erzielt.

So wird in der D3 ein einstufiges Hydrolyseverfahren mit Schwefelsäure als Verseifungsagenz publiziert, das ohne Lösungsmittel auskommt und direkt zu konzentrierten wässrigen MHA-Lösungen führt, wobei als Coprodukt kristallines Ammoniumsulfat in verkaufsfähiger Form erhalten wird. Dieses Ziel wird erreicht, indem man das Verseifungsgemisch mit Ammoniumhydroxidlösung soweit neutralisiert, daß die überschüssige Mineralsäure und das entstandene Ammoniumbisulfat in das neutrale Sulfat überführt werden, wobei zwei flüssige Phasen entstehen, die ihrerseits getrennt und eingedampft werden, um flüssiges MHA einerseits und kristallines Ammoniumsulfat andererseits zu gewinnen. Dabei werden die verschiedenen Filtrations- und Rückführungsschritte so kombiniert, daß praktisch kein Produkt verloren geht und kein mit Salz belastetes Abwasser entsteht. Das resultierende MHA ist von ähnlicher Qualität wie das nach der D1 erhaltene Produkt.

Jedoch auch dieses relativ umweltschonende Verfahren weist diverse Nachteile auf. Wie die Anmelderin der vorliegenden Erfindung beim Nacharbeiten dieses Verfahrens feststellte, müssen zum einen, bedingt durch die vergleichsweise höhere Verdünnung der Schwefelsäure (20 - 50 %), deutlich höhere Säureüberschüsse als angegeben verwendet werden, um zu einem vollständigen Cyanhydrin-Umsatz zu kommen. Auch muß zur Vermeidung von Salzausscheidungen während der Neutralisation in höherer Verdünnung gearbeitet werden, um die beiden flüssigen Phasen sauber trennen zu können. Zum anderen ist das isolierte Ammoniumsulfat von klebriger Konsistenz und mit einem intensiven Geruch behaftet, so daß eine Nachbehandlung wie z. B. eine Waschfiltration oder Umkristallisation unumgänglich erscheint, wodurch das Verfahren zusätzlich verteuert wird. Auch ist das Verfahren in den Eindampfschritten - anders als postuliert - energieaufwendiger als das zum Vergleich herangezogene Verfahren der D1. Kostenintensiv und apparativ sehr aufwendig ist außerdem das mit zwei getrennten Strängen ausgestattete Feststoffhandling mit Filtration/Zentrifugation sowie der im Fließdiagramm nicht angegebenen Trocknung des Ammoniumsulfats.

Einen teilweisen Ausweg aus dem Dilemma verspricht die D4. Offenbart wird in der D4 die Rückgewinnung von Schwefelsäure aus einem sulfathaltigen Abfallstrom, der bei der Herstellung von 2-Hydroxy-4-(methylthio)buttersäure durch Hydrolyse von 2-Hydroxy-4-(methylthio)butyronitril mit Schwefelsäure anfällt.

Die Rückgewinnung von Schwefelsäure aus Ammoniumsulfat-, Ammoniumbisulfat und/oder Schwefelsäurehaltigen Rückständen ist für die MMA-Herstellung schon lange Stand der Technik und wird genauso wie von den Rückständen der Verseifung des Acetocyanhydrins bekannt, durch Verbrennung der anfallenden Verseifungs- und Extraktionsabfallströme in einer sogenannten SK-Anlage (= Spalt-Kontakt-Anlage) erreicht.

Dabei wird in einer dem Fachmann geläufigen Weise zunächst $SO_2$ als Spaltprodukt erzeugt, welches am Kontakt zu $SO_3$ oxidiert wird, welches letztlich zur Schwefelsäure umgesetzt wird. Die resultierende Schwefelsäure kann dann wieder in den Verseifungsprozeß zurückgeführt werden, während sich die anderen ehemaligen Bestandteile der "Salzfracht" im wesentlichen in Form von Verbrennungsgasen finden lassen.

So elegant diese Methode auch sein mag, sie ist auch nicht frei von Nachteilen. So fallen bei den Verfahren

gemäß D1 und D2 Abfallströme an, deren Sulfatkonzentration relativ gering ist, in jedem Fall jedoch zu gering, um eine direkte Einspeisung in eine SK-Anlage zu gestatten. So ist eine Aufkonzentrierung oder Aufstockung durch Mischung mit konzentrierteren Abfallströmen aus anderen Prozessen i. d. R. unerläßlich. Übliche für den Betrieb von SK-Anlagen einzusetzende Lösungen weisen einen Sulfatsalzgehalt von > 50 Gew.-% auf. Höhere Konzentrationen sind noch mehr bevorzugt. Ein Aufkonzentrieren der MHA-Isolierungsabwässer durch Eindampfen ist jedoch aufgrund der hohen Korrosivität der Abwässer ein relativ aufwendiges Unterfangen, welches angefangen von einer speziellen Materialauswahl für die Eindampfungsaggregate bis hin zu besonderen Sicherheitsvorkehrungen aufwendig und teuer ist.

Angesichts des hierin angegebenen und diskutierten Standes der Technik sowie der mit den bekannten Verfahren verbundenen Nachteile ist es Aufgabe der Erfindung, ein weiteres Verfahren zur Herstellung von 2-Hydroxy-4-methylthiobuttersäure (MHA) gemäß der eingangs erwähnten Gattung anzugeben, welches hinsichtlich der Aufarbeitung der Reaktionsprodukte möglichst einfach und kostengünstig sein soll und ein möglichst hochkonzentriertes Produkt mit möglichst geringem Gehalt an Dimeren, Oligomeren und Nebenprodukten gestatten soll. Dabei soll das neue Verfahren nach Möglichkeit die Vorteile der einfachen Durchführbarkeit des MHA-Isolierungsschrittes in einer Flüssig/Flüssig-Extraktion beibehalten, gleichzeitig aber eine möglichst einfache und sinnvolle Entsorgung der anfallenden Salzfrachten in den sulfathaltigen Abwässern zulassen. Insbesondere soll ein Prozeß zur Verfügung gestellt werden, der dabei u. a. die direkte Einspeisung unmittelbarer Abfallströme in z. B. eine Spalt-Kontakt-Anlage zur Wiedergewinnung von einsatzfähiger und daher im Prozeß recyclierbarer Schwefelsäure gestattet.

Gelöst werden diese sowie weitere nicht im einzelnen angegebene Aufgaben mit einem Verfahren der eingangs genannten Gattung, welches das Merkmal des kennzeichnenden Teils des Anspruches 1 aufweist.

Vorteilhafte Verfahrensvarianten werden in den von Anspruch 1 abhängigen Verfahrensansprüchen unter Schutz gestellt.

Dadurch, daß der Salzgehalt des Reaktionsgemisches vor der Flüssig/Flüssig-Extraktion auf eine Konzentration von > 50 Gew.-%, bevorzugt > 55 Gew.-% (wt/wt), jeweils bezogen auf die Summe der nicht organischen Bestandteile des Reaktionsgemisches, gebracht wird, wird erfindungsgemäß ein Verfahren zur Verfügung gestellt, welches die Herstellung von flüssigem MHA in hervorragender Qualität gestattet und welches gleichzeitig in nicht ohne weiteres vorhersehbarer Weise die Problematik des zwangsweise anfallenden Salzes löst, zumindest jedoch überraschend verbessert. Insbesondere ist es ferner mit einer Reihe von erheblichen Vorteilen verbunden, die Salzkonzentration nach der Hydrolyse aber vor der Flüssig/Flüssig-Extraktion und damit in jedem Fall nicht unmittelbar vor der möglichen Kopplung mit einer SK-Anlage auf geeignete Weise anzuheben.

Zu den angesprochenen Vorteilen gehören u. a.:

- ♦ Die ansonsten vor Einspeisung in eine SK-Anlage fällige Eindampfung kann vollständig entfallen.
- ♦ Für eine Aufkonzentrierung vor der Extraktion sind verfahrenstechnisch einfachere und damit billigere Aggregate ausreichend, weil die aufzukonzentrierende Lösung aufgrund ihrer speziellen Zusammensetzung während dieses Verfahrenszeitpunktes sehr viel weniger aggressiv und damit insbesondere auch weniger korrosiv ist.
- ♦ Es ergibt sich im Fall der zumindest teilweisen Verschiebung der Eindampfung auf einen vorgezogenen Verfahrenszeitpunkt ein insgesamt verbesserter Energieverbund. Da bereits temperiertes (heißes) Hydrolysat eingedampft wird, und nicht bereits durch Extraktion abgekühltes Raffinat, ist der Energieaufwand geringer.
- ♦ Bei einer Aufkonzentrierung durch Eindampfung wird die Abtrennung von unerwünschten leichtsiedenden Bestandteilen des Hydrolysats verbessert.
- ♦ Die Hydrolyse des MHA-Amids kann verdünnter durchgeführt werden, woraus ein vollständigerer chemischer Umsatz resultiert. Da es bei der Hydrolyse des MHA-Amids mit Schwefelsäure besser ist, eine verdünntere Schwefelsäure (< 40 Gew.-%) einzusetzen, um die Hydrolyse möglichst vollständig ablaufen zu lassen, kann eine weniger stark konzentrierte Schwefelsäure in der Hydrolyse des MHA-Amids eingesetzt werden, ohne befürchten zu müssen, daß die anschließende Extraktion mit einem organischen Lösungsmittel ungünstiger verlaufen würde. Durch die Aufkonzentrierung wird eine Verschlechterung des Verteilungskoeffizienten vermieden, insbesondere verbleibt bei der Extraktion weniger MHA im Raffinat.

Damit erfüllt das erfindungsgemäße Verfahren insbesondere auch die Forderung nach einer vorteilhaften Schwefelsäurekonzentration bei der Verseifung sowie der Gewinnung von MHA aus dem Hydrolysat in Verbindung mit der Bereitstellung eines Raffinats, welches aufgrund seiner Zusammensetzung besser für eine Aufarbeitung in einer SK-Anlage geeignet ist, wobei gleichzeitig die Energiebilanz insgesamt erheblich verbessert wird.

Die Konzentration des Salzes im Hydrolysat von etwa > 50 Gew.-% stellt dabei eine für die spätere Aufarbeitung mittels einer SK-Anlage schon recht brauchbare Konzentration dar. Bevorzugt sind Bereiche von 55 bis 60 Gew.-%, ganz besonders zweckmäßig ist die Einstellung des Salzgehalts des Reaktionsgemisches auf etwa 60 bis 80 Gew.-%, jeweils bezogen auf die Summe der nicht organischen Bestandteile des Reaktionsgemisches. Für die Bestimmung dieses Wertes, der auch als Gehalt bezogen auf die "organic free basis" bezeichnet werden kann, werden im wesentlichen der Wassergehalt, Schwefelsäuregehalt und der Gehalt an Sulfat- bzw. Ammoniumionen herangezogen. Dies sind die wesentlichen nicht organischen Bestandteile des Hydrolysats.

Der Begriff Aufkonzentrierung erfaßt im Sinne der Erfindung allgemein die Erhöhung der Salzkonzentration (bezogen auf eine Basis frei von organischen Bestandteilen in Gew.-% = "organic free basis").

In einer bevorzugten erfindungsgemäßen Verfahrensvariante wird dem Reaktionsgemisch (Hydrolysat), das durch Anlagerung von HCN an MMP und Hydrolyse des entstandenen MMP-CH mit $H_2SO_4$ entstanden ist, zur Aufkonzentrierung eine geeignete Menge Ammoniumsulfat zugesetzt. Die Konzentration an Salz wird demnach durch einen Zusatz gesteigert. Speziell diese Variante beinhaltet mehrere große Vorteile.

Wie bereits erwähnt, sind prinzipiell zwei gegenläufige Effekte zu beachten. Einerseits soll die Hydrolysereaktion möglichst vollständig ablaufen können, wozu eine relativ geringere Amidkonzentration im vorhandenen Wasser bei der MHA-Amid-Verseifung geeignet erscheint. Hieraus resultiert zwangsläufig eine verdünnte Lösung aus MHA und Ammoniumhydrogensulfat. Andererseits ist es für die Extraktion günstiger, den Wasseranteil des Hydrolysats zu verringern, d. h. eine möglichst hohe MHA-Konzentration in der Wasserphase zu haben.

Gemäß der D1 und der D2 wird deshalb bei etwa < 40 Gew.-% Schwefelsäurekonzentration hydrolysiert, was jedoch zwangsläufig zu einer verdünnten Lösung aus MHA und Ammoniumhydrogensulfat führt. Um die anschließende Extraktion zu verbessern wird gemäß der D1/D2 das Ammoniumhydrogensulfat durch Zugabe von wasserfreiem Ammoniak in neutrales Ammoniumsulfat umgewandelt. Dies verbessert zwar das Korrosionsverhalten der Lösung, es kann jedoch zur Ausfällung von Feststoffen kommen, die den Betrieb einer Extraktion ungünstig beeinflussen können. Deshalb kann gemäß D1/D2 nachträglich wieder Wasser zugegeben werden, um die ausgefallenen Salze wieder in Lösung zu bringen. Es sollte aber dabei die Konzentration von MHA bzw. Salz nicht zu weit abgesenkt werden, da sonst wieder schlechter extrahiert werden kann.

Im Gegensatz dazu wird es durch die erfindungsgemäße Zugabe von Ammoniumsulfat möglich, die Hydrolyse des MHA-Amids verdünnter durchzuführen, was zu vollständigerem Umsatz in der Hydrolysestufe führt; gleichzeitig muß nicht neutralisiert werden. D.h. das Handling mit wasserfreiem Ammoniak und eine eventuelle Rückverdünnung sind nicht nötig.

In bevorzugter Ausführungsform wird das Verfahren der Erfindung so ausgeführt, daß vor Isolierung des MHA eine zum Aussalzen unter Zweiphasenbildung wirksame Menge Ammoniumsulfat zugefügt wird.

Es ist. zwar aus D1/D2 bekannt, daß die Anwesenheit einer hohen Salzkonzentration (am besten Ammoniumhydrogensulfat) das MHA "aussalzt" und somit einen günstigen Effekt auf die Verteilungskoeffizienten hat. Eine Zweiphasigkeit soll jedoch gemäß D1/D2 vermieden werden, da dies die Extraktion beeinträchtigen würde. Im Gegensatz hierzu wurde gefunden, daß eine Zweiphasigkeit durch Zugabe von Ammoniumsulfat besonders förderlich für den Extraktionsschritt ist und diesen insgesamt positiv beeinflußt. So kann z. B. in einer Variante zu einem erfindungsgemäß erhaltenen Zweiphasengemisch soviel Lösungsmittel gegeben werden, daß sich zwei klare Phasen bilden und nur einmal eine Trennung durchgeführt werden muß.

Darüber hinaus hat die Zugabe von Ammoniumsulfat an dieser Stelle des MHA-Gewinnungsverfahrens den allgemeinen Vorteil der Produktschonung. Durch Zugabe von Ammoniumsulfat wird nur einseitig die Ammoniumsalzkonzentration erhöht und nicht gleichzeitig die MHA-Konzentration. Eine weitere thermische Belastung (Verfärbung) des angestrebten Zielprodukts kann entfallen, im Gegenteil, durch das Auflösen des Ammoniumsulfats tritt eine Temperaturerniedrigung ein. Trotzdem wird der Verteilungskoeffizient günstig beeinflußt.

Obwohl die diskutierte Variante der Ammoniumsulfatzugabe unbestreitbare Vorteile bei der Produktschonung besitzt, kann es in einer alternativen Abwandlung des erfindungsgemäßen Verfahrens bevorzugt sein, die Salzkonzentration des Reaktionsgemisches (Hydrolysats) durch Eindampfung anzuheben.

Je nach Salzkonzentration im Raffinat kann es zu vermehrtem Sumpfaustrag von Lösungsmittel kommen, der gemäß den Druckschriften D1, D2 oder D4 in einer Raffinatstrippstufe abgetrennt werden muß. Im Gegensatz hierzu wurde erfindungsgemäß festgestellt, daß ein auf diese Weise zurückgewonnenes Lösungsmittel bedingt durch eine thermische Belastung in stark saurem Medium stark mit Nebenprodukten verunreinigt und somit für eine direkte Rückführung in das Extraktionssystem ungeeignet ist.

Überraschenderweise zeigte sich im Rahmen der Erfindung, daß bereits leichtes Kühlen des Sumpfproduktes zum Austrag eines zweiphasigen Gemisches von wäßrigem und organischem Raffinat führt, wobei das organische Raffinat zu > 97 % aus Lösungsmittel besteht, welches direkt nach einfacher Abscheidung in einem Trenngefäß ohne weiteren Zusatzaufwand in das Extraktionssystem zurückgeschleust und auf diese Weise der Lösungsmittelverlust minimiert werden kann.

Mithin kennzeichnet sich eine besonders bevorzugte Variante des erfindungsgemäßen Verfahrens, bei dem die Salzkonzentration durch Eindampfung angehoben wird, dadurch, daß unmittelbar aus dem Extraktionssystem wenigstens drei flüssige Phasen resultieren.

Hierbei ist zwar grundsätzlich die Möglichkeit gegeben, daß ein homogenes Raffinat und ein aus zwei flüssigen Phasen bestehender Extrakt gebildet werden, wobei das Extrakt als erste flüssige Phase im wesentlichen aus MHA, Lösungsmittel und geringen Teilen Wasser besteht, während es als zweite flüssige Phase im wesentlichen aus Wasser, MHA und geringen Teilen Salz besteht, es ist jedoch bei weitem vorteilhafter, daß bei einer erfindungsgemäßen Verfahrensvariante ein homogener Extrakt und ein aus zwei flüssigen Phasen bestehendes Raffinat gebildet wird. In diesem Falle ist es besonders zweckmäßig, wenn das Raffinat als erste flüssige Phase im wesentlichen aus Ammoniumsalz und Wasser und zu geringen Teilen aus MHA und organischem Lösungsmittel besteht, während es als zweite flüssige Phase im wesentlichen aus organischem Lösungsmittel und zu geringen Teilen aus Wasser und MHA besteht.

In vorteilhafter Abwandlung des Verfahrens der Erfindung werden dabei die an die eigentliche Eindampfung nachfolgenden Schritte so geführt, daß die zweite flüssige Phase MHA in einer Menge von 0,01 bis 0,5 Gew.-% enthält, Lösungsmittel in einer Menge von 90 bis 99 Gew.-% enthält und Wasser in einer Menge von 0,1 bis 10 Gew.-% enthält, während die erste flüssige Phase Wasser in einer Menge von 20 bis 50 Gew.-% enthält, MHA in einer Menge von 0,01 bis 0,5 Gew.-% enthält und Salz in einer Menge von 50 bis 80 Gew.-% enthält, wobei die Bestandteile jeder Phase für sich genommen 100 Gew.-% ergeben müssen.

Die für die Eindampfung zur Gewinnung des MHA verwendete Extraktionslösung wird aus dem Reaktionsgemisch durch Extraktion gewonnen. Grundsätzlich sind hierfür natürlich alle im Stand der Technik bekannten organischen Lösungsmittel einsetzbar, die eine Reihe bereits einleitend angegebener Eigenschaften aufweisen. Das zur Extraktion eingesetzte organische Lösungsmittel sollte im wesentlichen mit Wasser nicht mischbar sein. Allerdings ist eine partielle Mischbarkeit des organischen Lösungsmittels mit Wasser tolerierbar. Unter den für die Stofftrennung in der Flüssig/Flüssig-Extraktion in Frage kommenden Lösungsmitteln gibt es eine Vielzahl, die die Bedingungen der chemischen Indifferenz und einer geringen Löslichkeit für Wasser erfüllen. Im allgemeinen ist es bevorzugt, daß die Löslichkeit von Wasser im Lösungsmittel nicht größer als ca. 15 Gew.-%, vorzugsweise nicht größer als 10 Gew.-% bei Raumtemperatur ist. Unter den geeigneten Lösungsmitteln sind solche bevorzugt, die einen Siedepunkt von zwischen ca. 60 °C und ca. 200 °C, bevorzugt zwischen ca. 70 °C und 150 °C, aufweisen. Der Verteilungskoeffizient zwischen dem Lösungsmittel, welches das extrahierte MHA enthält, und dem wässrigen Raffinat, welches nach dem Kontaktieren von Lösungsmittel und MHA-Hydrolysat zurückbleibt, sollte mindestens bei ca. 2 für das MHA im Gleichgewicht liegen. Vorzugsweise ist dieser Verteilungskoeffizient wenigstens = 5. Auch soll der Verteilungskoeffizient für MHA im Gleichgewicht zwischen Extraktionslösung und Waschwasser ca. 1,0 nicht unterschreiten. Weiterhin soll das Lösungsmittel eine niedrige Toxizität aufweisen.

Eine Reihe von Ketonen, Aldehyden und Carbonsäureestern sind besonders als Lösungsmittel für die Extraktion geeignet. Besonders bevorzugte Lösungsmittel sind Ketone mit verhältnismäßig geringem Molekulargewicht, wie z. B. Methyl-n-propylketon, Methylethylketon, Methylamylketon, Methylisoamylketon, Methylisobutylketon, Ethylbutylketon und Diisobutylketon. Ebenfalls gut geeignete Lösungsmittel für die Extraktion sind Aldehyde, wie z. B. n-Butyraldehyd, und Ester, wie z. B. Ethylacetat, n-Butylacetat, n-Propylacetat und Isopropylacetat. Es können auch Alkohole verwendet werden, obwohl diese aufgrund ihrer wechselseitigen Löslichkeit mit Wasser, einer langsamen Phasenseparierung und der Tendenz mit dem MHA zu reagieren, weniger bevorzugt sind.

Gegenüber diesen bereits im Stand der Technik eingesetzten oder nahegelegten Lösungsmitteln, die selbstverständlich für die Erfindung auch eine gewisse Brauchbarkeit besitzen, hat es sich ganz überraschend herausgestellt, daß der Einsatz von Etherverbindungen als Lösungsmittel in der Extraktion große Vorteile mit sich bringt. Zu den erfindungsgemäß einsetzbaren Ethern gehören vor allem solche der allgemeinen Formel I

$$R^1{-}O{-}R^2 \qquad (I),$$

worin $R^1$ und $R^2$ unabhängig voneinander gleich oder verschieden $C_1$-$C_5$-Alkyl, linear oder verzweigt, sind.

Folgende Etherverbindungen kommen u.a. in Frage:

| Nr. | R1 | R2 | Sdp. [°C] |
|---|---|---|---|
| 1 | Ethyl | Ethyl | 35 |
| 2 | n-Propyl | Methyl | |
| 3 | n-Propyl | Ethyl | |
| 4 | n-Propyl | n-Propyl | 90 |
| 5 | i-Propyl | i-Propyl | 69 |

(fortgesetzt)

| Nr. | R1 | R2 | Sdp. [°C] |
|---|---|---|---|
| 6 | n-Butyl | Methyl | 71 |
| 7 | n-Butyl | Ethyl | 92 |
| 8 | n-Butyl | n-Propyl | |
| 9 | n-Butyl | n-Butyl | 143 |
| 10 | tert-Butyl | Methyl | 56 |
| 11 | tert-Butyl | Ethyl | 73 |
| 12 | tert-Butyl | n-Propyl | |
| 13 | tert-Butyl | n-Butyl | |
| 14 | Neopentyl | Methyl | |
| 15 | Neopentyl | Ethyl | |
| 16 | Neopentyl | n-Propyl | |
| 17 | Neopentyl | n-Butyl | |

Von den Etherverbindungen sind zum einen solche bevorzugt, die nicht oder nur wenig zur Bildung von Peroxiden neigen, wie z. B. MTBE. Bevorzugt sind auch asymmetrische Ether.

Zum anderen sind solche Verbindungen sehr zweckmäßig, die einen Siedepunkt < 60 °C aufweisen, da diese sich vollständig und einfach aus dem Zielprodukt entfernen lassen.

Ganz besonders günstig ist im Rahmen der Erfindung der Einsatz von Methyltertiärbutylether (MTBE), welches alle genannten Kriterien erfüllt.

Die Extraktion selbst kann grundsätzlich kontinuierlich oder intermittierend durchgeführt werden. Für eine diskontinuierliche Verfahrensweise eignet sich beispielsweise ein Rührkessel. Bevorzugt jedoch wird die Extraktion in einer kontinuierlichen Gegenstrom-Extraktionsanlage durchgeführt, welche eine zur Beschleunigung des Massentransfers zwischen Lösungsmittel und wäßriger Phase ausgebildete Extraktionszone aufweist. So ist es beispielsweise vorteilhaft, die Extraktion in einer Kaskade von kontinuierlichen Gegenstrom-Mischer-Abscheidern, einer Füllkörperkolonne, einer Siebbodenkolonne, bevorzugt als Pulsationskolonne oder Kolonne mit bewegten Sieben, einer Drehscheibenkolonne oder einem Zentrifugalextraktor durchzuführen. Bei einer besonders bevorzugten Ausführungsform wird die Extraktion in einer Siebbodenkolonne für Flüssig/Flüssig-Extraktion durchgeführt. Intermittierende oder impulsförmige Ströme, obwohl zyklisch also nicht kontinuierlich im Sinne von schneller Strömungsrate, werden im Zusammenhang mit der vorliegenden Offenbarung als "kontinuierlich" angesehen.

Der Extraktionsvorgang wird vorzugsweise reguliert, um die Lösungsmittelphase in der Extraktionszone als die kontinuierliche Phase herzustellen und aufrechtzuerhalten.

Um den Salzgehalt des Endproduktes auf ein Minimum zu bringen, wird der Extrakt ggf. mit Wasser gewaschen. In bestimmten Konzentrationsbereichen der resultierenden Extraktlösungen kann jedoch auf das Waschen verzichtet werden, insbesondere bei Sulfatgehalten von < 0,5 Gew.-% und im Hinblick auf die Salzkonzentration des ablaufenden Raffinats, die möglichst nicht weiter verdünnt werden sollte. In einem kontinuierlichen Gegenstromextraktionssystem kann der Extrakt durch Vermischen mit Wasser an einer Stelle stromaufwärts, in Bezug auf die Richtung der organischen Strömung, der Stelle, an der Hydrolysat in das Flüssig/Flüssigextraktionssystem eingebracht wird, gewaschen werden. So werden beispielsweise in einer vertikalen Kolonne unter Verwendung eines Lösungsmittels, dessen spezifisches Gewicht bevorzugt weniger als 1 beträgt, Lösungsmittel in die Kolonne an einer Stelle unterhalb der Zufuhrstelle, an der wässrige Hydrolysatlösung eingebracht wird, und Waschwasser in die Kolonne an einer Stelle oberhalb des Zufuhrpunktes der Hydrolysatlösung eingebracht.

Die Produktivität des Extraktionsvorganges wird durch Arbeiten bei einer etwas erhöhten Temperatur, um für die Lösungsmittelphase innerhalb des Extraktionssystems eine relativ niedrige Viskosität vorzusehen, erhöht. Das Arbeiten bei einer Temperatur im Bereich bis unterhalb des Siedepunktes des eingesetzten organischen Lösungsmittels ergibt auch eine gerade noch günstige Wirkung auf den MHA-Verteilungskoeffizienten zwischen der organischen und der wässerigen Phase.

Im Rahmen der Erfindung kann das MHA aus der Extraktionslösung wie bereits erwähnt durch Eindampfung gewonnen werden. D. h., die vorliegende Erfindung befaßt sich in einem weiteren Aspekt, insbesondere mit der Eindampfung einer Extraktionslösung, wie sie aus einer Flüssig-Flüssig-Extraktion eines Reaktionsgemisches erhältlich

ist, das beispielsweise durch Hydrolyse von MMP-CH mit Schwefelsäure erhalten wird. Dabei wird die Eindampfung bevorzugt so geführt, daß der verbleibende Extrakt weniger als 4 Gew.-%, bevorzugt weniger als 2 Gew.-%, Wasser aufweist. Hierbei ergibt sich in nicht ohne weiteres vorhersehbarer Weise ein hochkonzentriertes flüssiges MHA mit besonders niedrigem Oligomeren- und Dimerenanteil. Angesichts des bekannten Standes der Technik ist es mehr als überraschend, daß dies mit geringerem Wasseranteil erreicht werden kann, als etwa den bekannten Druckschriften (D1 und D2) entnehmbar ist.

In besonders bevorzugter erfindungsgemäßer Ausführungsform wird das organische Lösungsmittel bei der Eindampfung mit einem Aggregat entfernt, welches eine kurze Verweilzeit der Extraktionslösung in einer Eindampfungsstufe gestattet. Besonders bevorzugt wird das organische Lösungsmittel bei der Eindampfung daher mit einem Fallfilmverdampfer, Dünnschichtverdampfer und/oder Kurzwegverdampfer oder unter Mithilfe eines solchen Aggregats aus der Extraktionslösung abgetrennt.

Unter dem Begriff "Mithilfe eines solchen Aggregats" wird im Rahmen der Erfindung verstanden, daß die genannten Aggregate mit kurzer Verweilzeit der Extraktionslösung auch mit dem Fachmann bekannten Einrichtungen zur Abtrennung des Lösungsmittels aus Extraktionslösungen kombiniert werden können. Hierbei muß es sich bei den, zur Kombination verwendeten Aggregaten nicht unbedingt um solche, mit einer kurzen Verweilzeit handeln. Genannt werden können an dieser Stelle u. a. Destillationskolonnen, die wahlweise auch zur Einleitung von Dampf oder anderen geeigneten Strippmitteln ausgerüstet sein können. Auch Kombinationen, die mehrere der aufgeführten Aggregate mit kurzer Verweilzeit umfassen, sind möglich.

In vorteilhafter Abwandlung des erfindungsgemäßen Verfahrens ist es bevorzugt, die Eindampfung der Extraktionslösung so zu führen, daß ein möglichst geringer Restlösungsmittelgehalt eingestellt wird. Dies wird beispielsweise durch Kombination mehrerer oben genannter Aggregate mit einer Strippstufe, welche als zusätzliches Aggregat oder integriert in die vorgenannten Aggregate im Verdampfersystem enthalten sein kann, wie z. B. beim direkten Eintrag des Strippmediums in einen solchen Verdampfer, erreicht.

Die spezifischen Bedingungen für die Eindampfung variieren notwendigerweise mit dem für die Verwendung bei der Extraktion verwendeten speziellen Lösungsmittel. Grundsätzlich ist es für die Eindampfung unter Verwendung eines Abtrennaggregats mit kurzer Verweilzeit der Extraktionslösung bevorzugt, daß der Druck während der Eindampfung nicht mehr als 600 mbar, bevorzugt nicht mehr als 400 mbar und besonders bevorzugt nicht mehr als 200 mbar, beträgt.

Die bei der Eindampfung anzuwendende Temperatur ist im Regelfall ebenfalls vom abzutrennenden Lösungsmittel abhängig. Es wird jedoch angestrebt, und ist daher im Rahmen der Erfindung auch besonders bevorzugt, daß die Temperatur während der Eindampfung nicht höher als 150 °C ist. Wird diese Temperatur sehr deutlich überschritten, so kann es zur thermischen Schädigung des angestrebten Produktes kommen. Hierbei versteht sich unter der Temperatur während der Eindampfung nicht notwendigerweise die Kontakttemperatur des Produktes mit der Oberfläche des zur kurzzeitigen Berührung mit dem Produkt ausgestatteten Verdampfungsaggregates. Vielmehr ist mit der Temperatur während der Eindampfung die durchschnittliche Temperatur im Eindampfungsaggregat gemeint. Die Temperatur an der Oberfläche des Eindampfungsaggregates kann im Zweifelsfalle sehr viel höher liegen, als die 150 °C. Entscheidend ist die Kürze der Kontaktzeit bei den verwendeten Eindampfungsaggregaten. Hierdurch wird eine thermische Schädigung vermieden, selbst wenn die Kontakttemperatur deutlich über 150 °C liegen sollte.

Bezüglich der Temperaturverteilung hat es sich im Rahmen der Erfindung herausgestellt, daß es für die Produktqualität von besonderem Vorteil ist, wenn die Temperatur des verbleibenden Extraktes unmittelbar am Austritt aus dem Eindampfungsaggregat zwischen 30 und 100 °C, bevorzugt 50 bis 95 °C und besonders bevorzugt 70 bis 90 °C, beträgt.

Wie bereits erwähnt, ist die Verweilzeit des verbleibenden Extraktes mit ausschlaggebend für die Qualität und Zusammensetzung des angestrebten MHA-Produkts. In vorteilhafter Weiterbildung des erfindungsgemäßen Verfahrens beträgt die Verweilzeit des verbleibenden Extraktes in der Eindampfung nicht länger als 1,5 h. Dies bezieht sich auf die Verweilzeit im gesamten Eindampfungssystem, welches zumindest eine Eindampfungsstufe mit sehr kurzer Verweilzeit aufweist. Die Verweilzeit im Aggregat mit sehr kurzer Verweilzeit ist entgegen der für die Gesamtverweilzeit maximal angegebenen 1,5 h eher im Minutenbereich oder darunter anzusiedeln. Auf jeden Fall ist es im Rahmen der Erfindung bevorzugt, für den Fall, daß die Eindampfung lediglich aus einem Dünnschichtverdampfer und/oder Fallfilmverdampfer und/oder Kurzwegverdampfer besteht, daß die Verweilzeit in diesen Aggregaten nicht länger als 1 h, bevorzugt 40 min beträgt.

In einem weiteren Aspekt verbessert das erfindungsgemäße Verfahren zusätzlich zur Isolierung des MHA's aus dem durch Hydrolyse mit Schwefelsäure erhaltenen Reaktionsgemisch auch die Hydrolyse des MMP-CH's selbst. So wird die Hydrolyse des MMP-CH so geführt, daß in einer ersten Stufe das MMP-CH mit 60 - 85 gew.-%iger, bevorzugt mit 65 - 80 gew.-%iger Schwefelsäure im Molverhältnis MMP-CH zu $H_2SO_4$ von 1,0:0,5 bis 1:1,0, vorzugsweise 1:0,6 bis 1:0,95 bei Temperaturen von 30 - 90 °C, vorzugsweise 50 - 70 °C unter Erhalt von im wesentlichen MHA-Amid hydrolysiert wird. Hierbei entsteht im wesentlichen aus dem MMP-Cyanhydrin das MHA-Amid, wobei die entstehende Mischung weiterhin im wesentlichen frei von nicht umgesetzten MMP-Cyanhydrin ist. Mit anderen Worten bedeutet

dies, daß die Hydrolyse nahezu quantitativ verläuft. Das MHA-Amid wird in einer zweiten Stufe unter Zugabe von Wasser ohne weitere Zugabe von $H_2SO_4$ bei Temperaturen bis 140°C hydrolysiert.

Mit den hierin weiter oben beschrieben Abwandlungen der MHA-Gewinnung ist ein besonders vorteilhaftes Zielprodukt isolierbar. Das verbesserte MHA kennzeichnet sich erfindungsgemäß dadurch, daß es mehr als 95 Gew.-% MHA-Gesamt als Summe von monomerem MHA, MHA-Dimerem und MHA-Oligomeren (= MHA-Gesamt) sowie zwischen mehr als 0,1 und weniger als 5 Gew.-% Wasser aufweist. Insbesondere hat es sich für die Erfindung als vorteilhaft erwiesen, daß ohne größere Qualitätseinbußen ein MHA erhalten werden kann, welches sich dadurch kennzeichnet, daß es mehr als 98 Gew.-% MHA als Summe von monomerem MHA, MHA-Dimerem und MHA-Oligomeren sowie einen Wassergehalt von zwischen 0,1 und weniger als 2 Gew.-% aufweist und eine kinematische Viskosität von > 100 $mm^2/s$ bei 25 °C. Dabei hat sich überraschenderweise herausgestellt, daß die kinematische Viskosität, nach Cannon-Fenske gemessen, von Hochkonzentrat (also MHA von zumindest 98 Gew.-% Wirkstoffgehalt) nach Lagerung und Verdünnung vergleichbar ist der kinematischen Viskosität eines 88 gew.-%igen Produktes. Trotz eines sich beim Hochkonzentrat nach einer Lagerung von ca. 300 Tagen bei Raumtemperatur einstellenden relativ hohen Dimeren- und Oligomerengehaltes von ca. 50 Gew.-% entspricht bei Verdünnung des abgelagerten Hochkonzentrates mit Wasser auf ca. 88 Gew.-% dessen kinematische Viskosität der des 88 gew.-%igen Handelproduktes, welches in entsprechenden Lagerversuchen lediglich eine Gleichgewichtskonzentration von nur ca. 25 Gew.-% an Dimeren und Oligomeren aufwies. In beiden Fällen, sowohl beim verdünnten Hochkonzentrat als auch beim Handelsprodukt, war der Gleichgewichtszustand erreicht. Diese Tatsache ist sehr überraschend und erweist sich als großer Vorteil für eine erfindungsgemäß hergestellte Hochkonzentrat-MHA-Variante. Im Hinblick darauf,daß dimere und oligomere Anteile die Viskosität des MHA's im allgemeinen nachteilig für die praktische Verarbeitung beeinträchtigen, war es um so überraschender, daß trotz hoher Ausgangsgehalte beim sogenannten Hochkonzentrat ein leicht pump- und damit transportierbares Gemisch mit günstiger Viskosität erhaltbar ist. Dies hat vielfältige Vorteile, insbesondere führen Viskosität und vor allem der hohe Wirkstoffgehalt dazu, daß das Hochkonzentrat wirtschaftlicher transportiert werden kann, da weniger Wasser transportiert wird, und trotzdem kann am Zielort in der Futtermühle auf handelsübliche Konzentrationen mit Wasser verdünnt werden, ohne ungünstige höhere Viskositäten hinnehmen zu müssen.

Ferner hat es sich im Rahmen der Erfindung herausgestellt, daß ein qualitativ besonders hochwertiges MHA bei geeigneter Führung der Hydrolysereaktion in Kombination mit der erfindungsgemäß einzusetzenden schonenden Eindampfung mit sehr kurzer Verweilzeit erhältlich ist. Das besonders vorteilhaft herstellbare MHA kennzeichnet sich vor allem durch einen Anteil der Summe aus Dimeren und Oligomeren bezogen auf die Gesamtsumme MHA von < 10 Mol %, bevorzugt < 7 Mol %. Dies bedeutet, daß entgegen dem im Stand der Technik verbreiteten Vorurteil, ein hochkonzentriertes MHA erhältlich ist, welches durch einen äußerst geringen Anteil an Dimeren und Oligomeren eine gut geeignete kurzzeitige Transportform darstellt. Für eine längere Transportdauer ist es dann bevorzugt, sich in Abhängigkeit von der Lagerzeit in zunehmendem Maße bildende Dimere und Oligomere durch Zugabe von Wasser und Einwirkung erhöhter Temperatur wieder in monomeres MHA zu überführen.

Weiterhin ist es im Rahmen der Erfindung möglich, das hochkonzentrierte MHA-Produkt zur Herstellung von Tierfuttermittelsupplementen einzusetzen. Dabei zeigt es sich, daß durch Abmischung des MHA-Konzentrats mit Wasser, Methionin und/oder Salzen des MHA's (bevorzugt Ammonium-MHA) (ggf. NH3 zur Erzeugung von NH4-MHA) alle grundsätzlich vom Markt benötigten nutritiven Wertigkeiten ohne Qualitätsverlust herstellbar sind.

Vor allen Dingen hat es sich äußerst überraschend bei Durchführung der Erfindung herausgestellt, daß die Abmischungen nicht nur durch Zugabe von geeigneten Mischungskomponenten wie Wasser, Methionin und/oder Ammonium-MHA ab Austritt des angestrebten MHA-Produkts aus der Eindampfungsstufe zugänglich sind, sondern daß ebenso und weiterhin besonders vorteilhaft für den Fall der Abmischung mit Ammonium-MHA direkt Ammoniak in das MHA-Produkt aus der Eindampfung eingeleitet werden kann. Hierbei wird in Abhängigkeit von der zugefügten Ammoniak-Menge ein gewünschter Anteil des MHA in Ammonium-MHA umgewandelt.

Nachfolgend wird die Erfindung unter Bezugnahme auf die beigefügten Figuren weitergehend erläutert. In den Figuren zeigen:

Figur 1 ein Fließdiagramm zur MHA-Isolierung nach Salzabtrennung und Flüssig/Flüssig-Phasentrennung im MHA-Hydrolysat, wobei die Wege 1), 2) und 3) unabhängig voneinander gegangen werden können;

Figur 2 ebenfalls ein schematisches Fließbild einer Ausführungsform des erfindungsgemäßen Verfahrens, bei dem eine Salzabtrennung ohne Flüssig/Flüssig-Phasentrennung vorgesehen ist und wobei auch hier die Wege 1) und 2) voneinander unabhängig sind;

Figur 3 eine schematische Darstellung eines Fließbildes zu einer weiteren erfindungsgemäßen Ausführungsform, bei welcher MHA ohne Salzabtrennung isoliert wird;

Figur 4 ein Fließbildschema noch einer weiteren Ausführungsform der Erfindung, bei der MHA nach Aufstockung

des Salzgehaltes gewonnen wird; und

Figur 5 eine schematische Darstellung einer zur Durchführung des Verfahrens der Erfindung geeigneten Apparatur;

Figur 6 eine schematische Darstellung einer weiteren zur Durchführung des Verfahrens der Erfindung geeigneten Apparatur.

In einer in Figur 1 gezeigten Verfahrensvariante wird MMP-Cyanhydrin (MMP-CH) in einer zweistufigen Hydrolysereaktion mit wäßriger Schwefelsäure zur methioninhydroxyanalogen Säure (MHA) umgesetzt. Das entstandene primäre MHA-Hydrolysat wird anschließend ausgehend von einer Konzentration von < 40 Gew.% MHA auf eine Konzentration von > 40 Gew.% bevorzugt > 45 Gew.% MHA eingedampft, so daß zwei flüssige Phasen entstehen.

Das bei der Eindampfung erhaltene Wasser wird kondensiert und der Hydrolysestufe wiederzugeführt, wobei die Kondensationstemperatur aus energieökonomischen Gründen möglichst nahe an der Temperatur gehalten wird, bei der die Hydrolyse stattfindet. Die dabei erhaltene Fraktion an Leichtsiedern (LS) mit unangenehmen Geruchseigenschaften wird vom Wasserdampf weitgehend abgetrennt, über Kopf entfernt, ggf. mit Unterstützung von Strippgasen wie z.B. Luft und vorzugsweise direkt ohne vorherige Kondensation einem Verbrennungsofen zugeführt. Dieser kann auch Bestandteil einer Anlage zur Wiedergewinnung von Schwefelsäure sein (sog. SK-Anlage).

Die beiden aus dem Sumpf der Eindampfung erhaltenen Flüssigphasen werden bei einer Temperatur, die > RT, aber maximal bei der Temperatur der Eindampfung liegt, voneinander getrennt.

Die untere wäßrige, überwiegend das entstandene Ammoniumsalz enthaltende Phase wird soweit abgekühlt bis ein wesentlicher Teil der gelösten Salze kristallisiert. (Weg 1) bzw.2)) Die dafür benötigte Temperatur liegt unter 30 °C. Das erhaltene Salzkristallisat wird durch Zentrifugation oder Filtration von der überstehenden Lösung abgetrennt. Das Salzkristallisat kann zur Entfernung noch anhaftenden Wertstoffs (MHA) mit einem geigneten organischen Lösungsmittel, aber auch mit Wasser oder einer wäßrigen Salzlösung gewaschen werden.

Die obere überwiegend MHA enthaltende organische Phase , sowie das wäßrige Filtrat und ggf. das organische Filtrat werden getrennt oder auch gemeinsam nach teilweiser oder vollständiger vorheriger Vermischung einem Flüssig/Flüssig-Extraktionssystem zugeführt (Weg 1) bzw.2)) und mit einem organischen Lösungsmittel in mindestens 2 Phasen zerlegt, nämlich in mindestens eine überwiegend organische Extraktlösung, die das Lösungsmittel und MHA sowie geringe Anteile Wasser und Salz enthält , bzw. ein wäßriges Raffinat, das überwiegend aus Salz und Wasser besteht und das anschließend vorzugsweise einer Anlage zur Wiedergewinnung von Schwefelsäure zugeführt wird (Weg 1)) und gegebenenfalls darüberhinaus ein organisches Raffinat, das überwiegend aus Lösungsmittel und geringen Teilen MHA bzw. Wasser besteht und das ins Extraktionssystem zurückgeführt werden kann.

Die organische Extraktlösung wird einem System zur Extrakteindampfung zugeführt, wobei das verdampfte Lösungsmittel und ggf. entsprechende Wasseranteile durch Kondensation wiedergewonnen und in die Extraktionsstufe zurückgeführt werden. Das dabei als Sumpfablauf der Eindampfung erhaltene MHA-Hochkonzentrat wird in einer Konditionierung durch Zugabe gewünschter Mengen Wasser und oder entsprechender Zuschlagstoffe wie z.B. Methionin oder $MHANH_4$-Salz auf die gewünschte MHA-Konzentration vorzugsweise zwischen 78 und 98 Gew.% eingestellt.

Das Salzkristallisat kann nach ggf. erfolgter Salzwäsche einer Aufreinigungs- bzw. Konditionierungsstufe zugeführt werden (Weg 1)), in der durch Zugabe entsprechender Mengen $NH_3$ und anschließender Kristallisation und Trocknung verkaufsfähiges Ammoniumsulfat erzeugt wird, oder aber als Rohware direkt der Trocknung zugeführt werden. Das Salzkristallisat kann aber auch, insbesondere nach Auflösung in Wasser als > 60 %ige konzentrierte Lösung einer Anlage zur Wiedergewinnung von Schwefelsäure zugeführt werden (Weg 2)). Besonders vorteilhaft dabei ist es, das filterfeuchte Salzkristallisat im Raffinat der Extraktionsstufe aufzulösen und die dabei erhaltene hochkonzentrierte Salzlösung von > 75 Gew.% Salzgehalt der Schwefelsäurerückgewinnung zuzuführen, weil dafür ein Salzgehalt von mindestens 60 Gew.% erforderlich ist und darüberhinaus jede weitere Konzentrationserhöhung zu einer Verbesserung der Energiebilanz einer solchen Anlage beiträgt. Die Aufkonzentration ist hier insbesondere ohne eine energieintensive Eindampfung der aus dem Verfahren erhältlichen Salzlösung möglich. Die so wiedergewonnene Schwefelsäure kann ganz oder teilweise der MHA-Hydrolysestufe wiederzugeführt werden.

Es kann aber auch von Vorteil sein, die wäßrige Phase ohne Salzabtrennung direkt einer Anlage zur Wiedergewinnung von Schwefelsäure zuzuführen, gemeinsam mit dem Raffinat der Extraktion (Weg 3)). Auch hierbei liegt der Salzgehalt vorteilhafterweise deutlich über 60 Gew.%. Dabei fällt ein Verlust von ca. 2,5 % d.Th. an MHA an, welches sich noch in der wäßrigen Phase gelöst befindet. Ein wesentlicher Vorteil dabei ist jedoch die starke Entlastung der Extraktions- bzw. der Eindampfungsstufe, da der Eingangsstrom zur Extraktion und damit auch der Lösungsmitteleinsatz nahezu halbiert werden kann im Gegensatz zu herkömmlichen Verfahren (vgl. D2), womit eine drastische Energieeinsparung insbesondere im Hinblick auf die Lösungsmitteleindampfung bzw. -Kondensation verbunden ist.

In einer in Figur 2 dargestellten zweistufigen Hydrolysereaktion wird MMP-Cyanhydrin (MMP-CH) mit wäßriger Schwefelsäure zur methioninhydroxyanalogen Säure (MHA) umgesetzt. Das entstandene primäre MHA-Hydrolysat wird anschließend ausgehend von einer Konzentration von < 40 Gew.% MHA auf eine Konzentration von >

40 Gew.%, bevorzugt > 45 Gew.% MHA eingedampft, so daß zwei flüssige Phasen entstehen.

Das bei der Eindampfung erhaltene Wasser wird kondensiert und der Hydrolysestufe wiederzugeführt, wobei die Kondensationstemperatur aus energieökonomischen Gründen möglichst nahe an der Temperatur gehalten wird, bei der die Hydrolyse stattfindet. Die dabei erhaltene Fraktion an Leichtsiedern (LS) mit unangenehmen Geruchseigenschaften wird vom Wasserdampf weitgehend abgetrennt über Kopf entfernt, ggf. mit Unterstützung von Strippgasen wie z.B. Luft und vorzugsweise direkt ohne vorherige Kondensation einem Verbrennungsofen zugeführt. Dieser kann auch Bestandteil einer Anlage zur Wiedergewinnung von Schwefelsäure sein.

Die beiden aus dem Sumpf der Eindampfung erhaltenen Flüssigphasen werden gemeinsam mindestens soweit abgekühlt bis eine Suspension aus Salzkristallisat und einer homogenen organisch-wäßrigen Flüssigphase entsteht. Dabei ist es vorteilhaft bis auf Raumtemperatur abzukühlen.

Das Salzkristallisat wird durch Zentrifugation oder Filtration von der überstehenden Flüssigphase abgetrennt. Das Salzkristallisat wird zur Entfernung noch anhaftenden Wertstoffs (MHA) mit einem geigneten organischen Lösungsmittel oder auch mit Wasser oder einer wäßrigen Salzlösung gewaschen.

Das Filtrat und ggf. das organische Filtrat werden getrennt oder auch gemeinsam nach teilweiser oder vollständiger vorheriger Vermischung einem Flüssig/Flüssig-Extraktionssystem zugeführt und mit einem organischen Lösungsmittel in mindestens 2 Phasen zerlegt, nämlich in mindestens eine überwiegend organische Extraktlösung, die das Lösungsmittel und MHA sowie geringe Anteile Wasser und Salz enthält , bzw. ein wäßriges Raffinat, das überwiegend aus Salz und Wasser besteht und das anschließend vorzugsweise einer Anlage zur Wiedergewinnung von Schwefelsäure zugeführt wird (Weg 1)).

Die organische Extraktlösung wird einem System zur Extrakteindampfung zugeführt, wobei das verdampfte Lösungsmittel und ggf. entsprechende Wasseranteile durch Kondensation wiedergewonnen und in die Extraktionsstufe zurückgeführt werden. Das dabei als Sumpfablauf der Eindampfung erhaltene MHA-Hochkonzentrat wird in einer Konditionierung durch Zugabe gewünschter Mengen Wasser und oder entsprechender Zuschlagstoffe wie z.B. Methionin oder $MHANH_4$-Salz auf die gewünschte MHA-Konzentration vorzugsweise zwischen 78 und 98 Gew.% eingestellt.

Das Salzkristallisat kann nach ggf. erfolgter Salzwäsche einer Aufreinigungs- bzw. Konditionierungsstufe zugeführt werden (Weg 1)), in der durch Zugabe entsprechender Mengen $NH_3$ und anschließender Kristallisation und Trocknung verkaufsfähiges Ammoniumsulfat erzeugt wird, oder aber auch als Rohware direkt der Trocknung zugeführt werden.

Das Salzkristallisat kann aber auch insbesondere nach Auflösung in Wasser als > 60 %ige konzentrierte Lösung einer Anlage zur Wiedergewinnung von Schwefelsäure zugeführt werden (Weg 2)). Besonders vorteilhaft hierbei ist es wieder, das filterfeuchte Salzkristallisat im Raffinat der Extraktionsstufe aufzulösen und die dabei erhaltene hochkonzentrierte Salzlösung von > 75 Gew.% Salzgehalt der Schwefelsäurerückgewinnung zuzuführen, weil dafür ein Salzgehalt von mindestens 60 Gew.% erforderlich ist und darüberhinaus jede weitere Konzentrationserhöhung zu einer Verbesserung der Energiebilanz einer solchen Anlage beiträgt. Die Aufkonzentration ist hier insbesondere ohne eine energieintensive Eindampfung der aus dem Verfahren erhältlichen Salzlösung möglich. Die so wiedergewonnene Schwefelsäure kann ganz oder teilweise der MHA-Hydrolysestufe wiederzugeführt werden.

Bei der in Figur 3 dargestellten Verfahrensvariante wird MMP-Cyanhydrin (MMP-CH) in einer zweistufigen Hydrolysereaktion mit wäßriger Schwefelsäure zur methioninhydroxyanalogen Säure (MHA) umgesetzt. Das entstandene primäre MHA-Hydrolysat wird anschließend ausgehend von einer Konzentration von < 40 Gew.% MHA auf eine Konzentration von > 40 Gew.%, bevorzugt > 45 Gew.% MHA eingedampft, so daß zwei flüssige Phasen entstehen.

Das bei der Eindampfung erhaltene Wasser wird kondensiert und der Hydrolysestufe wiederzugeführt, wobei die Kondensationstemperatur aus energieökonomischen Gründen möglichst nahe an der Temperatur gehalten wird, bei der die Hydrolyse stattfindet. Die dabei erhaltene Fraktion an Leichtsiedern (LS) mit unangenehmen Geruchseigenschaften wird vom Wasserdampf weitgehend abgetrennt über Kopf entfernt, ggf. mit Unterstützung von Strippgasen wie z.B. Luft und vorzugsweise direkt ohne vorherige Kondensation einem Verbrennungsofen zugeführt. Dieser kann auch Bestandteil einer Anlage zur Wiedergewinnung von Schwefelsäure sein (z. B. SK-Anlage).

Die beiden aus dem Sumpf der Eindampfung erhaltenen Flüssigphasen werden ggf. gemeinsam abgekühlt, jedoch nur soweit daß sich kein Salzkristallisat bildet.

Das Produkt der Eindampfung wird einem Flüssig/Flüssig-Extraktionssystem zugeführt und mit einem organischen Lösungsmittel in mindestens 2 Phasen zerlegt, nämlich in mindestens eine überwiegend organische Extraktlösung, die das Lösungsmittel und MHA sowie geringe Anteile Wasser und Salz enthält , bzw. ein wäßriges Raffinat, das überwiegend aus Salz und Wasser besteht und das anschließend vorzugsweise einer Anlage zur Wiedergewinnung von Schwefelsäure zugeführt wird. Die dabei gewünschte Salzkonzentration von mindestens 60 Gew% hängt ganz wesentlich vom Grad der Eindampfung des primären Hydrolysates ab. Dabei ist allerdings zu beachten, daß dieser nur so groß sein darf, daß sich keine Salzkristallisate innerhalb des Extraktionssystems durch zu starkes Aufkonzentrieren bilden. Die damit erreichbaren Salzkonzentrationen sind somit geringer als bei den in den Figuren 1 bzw. 2 gezeigten Verfahrensabläufen. Die so wiedergewonnene Schwefelsäure kann ganz oder teilweise der MHA-Hydrolysestufe wiederzugeführt werden.

Die organische Extraktlösung wird einem System zur Extrakteindampfung zugeführt, wobei das verdampfte Lösungsmittel und ggf. entsprechende Wasseranteile durch Kondensation wiedergewonnen und in die Extraktionsstufe zurückgeführt werden. Das dabei als Sumpfablauf der Eindampfung erhaltene MHA-Hochkonzentrat wird in einer Konditionierung durch Zugabe gewünschter Mengen Wasser und oder entsprechender Zuschlagstoffe wie z.B. Methionin oder $MHANH_4$-Salz auf die gewünschte MHA-Konzentration vorzugsweise zwischen 78 und 98 Gew.% eingestellt.

In der in Figur 4 dargestellten Variante wird MMP-Cyanhydrin (MMP-CH) in einer zweistufigen Hydrolysereaktion mit wäßriger Schwefelsäure zur methioninhydroxyanalogen Säure (MHA) umgesetzt. Das entstandene primäre MHA-Hydrolysat mit einer Konzentration von < 40 Gew.% MHA wird anschließend einer Verdampfungskühlung unterzogen, bei der die Temperatur ausgehend von einer Reaktionstemperatur von > 100 °C auf eine geeignet niedrigere Temperatur von z.B. 60 °C abgesenkt wird und gleichzeitig eine Fraktion an Leichtsiedern (LS) mit unangenehmen Geruchseigenschaften zusammen mit geringen Mengen an Wasserdampf destillativ vorzugsweise unter Anlegen eines Vakuums und ggf. mit Unterstützung von Strippgasen wie z. B. Luft abgetrennt wird und die direkt ohne vorherige Kondensation einem Verbrennungsofen zugeführt werden kann. Dieser kann auch Bestandteil einer Anlage zur Wiedergewinnung von Schwefelsäure sein.

Durch anschließende Zugabe von $(NH_4)_2SO_4$ und/oder $NH_4HSO_4$ zur homogenen MHA-Hydrolysatlösung wird die darin erhaltene Salzkonzentration soweit erhöht bis zwei flüssige Phasen entstehen und dabei jedoch kein wesentlicher Teil an ungelösten Feststoffen zurückbleibt.

Die beiden Flüssigphasen werden bei einer Temperatur > RT voneinander getrennt (Weg 1)). Die obere überwiegend MHA enthaltende organische Phase wird einem Flüssig/Flüssig-Extraktionssystem zugeführt (Weg 1)) und mit einem organischen Lösungsmittel in mindestens 2 Phasen zerlegt, nämlich in mindestens eine überwiegend organische Extraktlösung, die das Lösungsmittel und MHA sowie geringe Anteile Wasser und Salz enthält bzw. ein wäßriges Raffinat, das überwiegend aus Salz und Wasser besteht.

Das Raffinat wird anschließend vorzugsweise zusammen mit der unteren, wäßrigen, überwiegend das entstandene Ammoniumsalz enthaltenden Phase aus der Flüssig/Flüssig-Phasentrennung einer Anlage zur Wiedergewinnung von Schwefelsäure zugeführt (Weg 1)).

Dabei ergibt sich ein Verlust von ca. 2,5 % d.Th. an MHA, welches sich noch in der wäßrigen Phase gelöst befindet.

Ein wesentlicher Vorteil dabei ist die starke Entlastung der Extraktions- bzw. der Eindampfungsstufe, da der Eingangsstrom zur Extraktion und damit auch der Lösungsmitteleinsatz deutlich herabgesetzt werden kann, im Gegensatz zu herkömmlichen Verfahren (vgl. D2), womit eine drastische Energieeinsparung insbesondere im Hinblick auf die anschließende Lösungsmitteleindampfung bzw. - Kondensation verbunden ist.

Die organische Extraktlösung wird einem System zur Extrakteindampfung zugeführt, wobei das verdampfte Lösungsmittel und ggf. entsprechende Wasseranteile durch Kondensation wiedergewonnen und in die Extraktionsstufe zurückgeführt werden. Das dabei als Sumpfablauf der Eindampfung erhaltene MHA-Hochkonzentrat wird in einer Konditionierung durch Zugabe gewünschter Mengen Wasser und oder entsprechender Zuschlagstoffe wie z.B. Methionin oder $MHANH_4$-Salz auf die gewünschte MHA-Konzentration vorzugsweise zwischen 78 und 98 Gew.% eingestellt.

Alternativ können auch beide Flüssigphasen gemeinsam einem Flüssig/Flüssig-Extraktionssystem zugeführt werden (Weg 2)). Das dabei entstehende Raffinat, eine > 60 %ige konzentrierte Salzlösung kann direkt einer Anlage zur Wiedergewinnung von Schwefelsäure zugeführt werden (Weg 2)). weil dafür ein Salzgehalt von mindestens 60 Gew.% erforderlich ist und darüberhinaus jede weitere Konzentrationserhöhung zu einer Verbesserung der Energiebilanz einer solchen Anlage beiträgt. Die Aufkonzentration ist hier insbesondere ohne eine energieintensive Eindampfung der aus dem Verfahren erhältlichen Salzlösung möglich, was einen großen Vorteil darstellt. Die so wiedergewonnene Schwefelsäure kann ganz oder teilweise der MHA-Hydrolysestufe wiederzugeführt werden.

Folgende präparativen Beispiele verdeutlichen den Gegenstand der Erfindung weiter:

Analytische Bestimmungsmethoden und Definitionen

Die Gehalte an MMP-Cyanhydrin, MHA-Amid bzw. MHA-Monomerem wurden in den Prozeßlösungen quantitativ per HPLC durch Vergleich mit einem externen Standard (Reinsubstanz) bestimmt.

Der Gehalt an MHA-Gesamt = MHA-Amid (ggf.) + MHA-Monomeres (= MHAges) + MHA-(Dimere + Oligomere)

wurde durch titrimetrische Bestimmung der Thioether-Funktion mit $KBr/KBrO_3$-Maßlösung bestimmt und als Summe der entsprechenden MHA-Monomeräquivalente in [Gew%] bzw. [g] bzw. [mol] bzw. [Mol%] ausgedrückt.

Der Gehalt an MHA-Dimere + MHA-Oligomere (DIM + OLI) wurde durch Berechnung der Differenz aus MHA-Gesamt und MHA-Monomer (+ ggf. MHA-Amid) ermittelt und als Summe der entsprechenden MHA-Monomeräquivalente in [Gew%] bzw. [g] bzw. [mol] bzw. [Mol%] ausgedrückt.

Der Wassergehalt wurde per Titration nach Karl-Fischer, der Lösungsmittel-Gehalt per GC oder Differenzbildung, der Sulfat- bzw. Ammonium-Gehalt per Ionenchromatographie nach Standard-Verfahren und der Gesamtsalzgehalt durch Umrechnung der Sulftat- bzw. Ammoniumgehalte oder durch Differenzbildung bestimmt.

Beispiel 1: Kontinuierliche Herstellung von MHA-Hydrolysatlösung

In einer 2-stufigen Rührkesselkaskade wurde durch kontinuierliche Einspeisung von 4,2 kg/h (31,3 mol/h) 97,7 % MMP-Cyanhydrin und 4,5 kg/h (29,7 mol/h) 65 % wäßrige $H_2SO_4$ bei einer Temperatur von 50 °C und einer mittleren Gesamtverweilzeit von 60 min insgesamt 8,7 kg/h einer MHA-Amidlösung hergestellt. Die MHA-Amidlösung wurde nach kontinuierlicher Verdünnung mit 3,6 kg/h Wasser in einer 2-stufigen Rührkesselkaskade mit nachgeschaltetem Reaktionsrohr bei einer Temperatur von 90 - 110 °C und einer mittleren Gesamtverweilzeit von 180 min zu 12,3 kg/h MHA-Hydrolysatlösung weiter umgesetzt. Die primär anfallende Reaktionslösung wurde durch kontinuierlichen Eintrag in ein Verdampfungssystem bei einem Druck von 100 mbar eingeengt und dabei auf eine Temperatur von 50 °C im Ablauf abgekühlt. Das dabei angefallene voreingedampfte MHA-Hydrolysat (10,8 kg/h) hatte die folgende analytische Zusammensetzung:

43,7 Gew.-% MHAges.
26,5 Gew.-% $SO_4^{2-}$ } ≙ 32,0 Gew.-% Salz
5,2 Gew.-% $NH_4^+$ }
24,3 Gew.-% $H_2O$

Beispiel 2: Herstellung von MHA-MTBE-Extraktlösung

Versuch 1

In einem 5-1-Rührbehälter mit Bodenablaßventil wurden 2,5 kg MHA-Hydrolysat (43,7 Gew% MHAges, hergestellt wie unter Beispiel 1 beschrieben) mit 1,5 kg MTBE (technisch) versetzt und 10 min bei RT intensiv gerührt. Nach Beenden des Rührens wurden die beiden entstandenen flüssigen Phasen voneinander getrennt. Der Vorgang wurde insgesamt 4 mal mit frischen Lösungen wiederholt.

Die organischen Phasen bzw. die wäßrigen Raffinatphasen wurden jeweils vereinigt und analysiert. Die Zusammensetzungen der Phasen in [Gew.%] werden in der nachfolgenden Tabelle 1 wiedergegeben.

Tabelle 1

| | organische Phase (13,0 kg) [Gew.-%] | Raffinat-Phase (7,0 kg) [Gew.-%] |
|---|---|---|
| MHAges | 41,8 | 1,9 |
| MHA | 38,4 | 1,9 |
| DIM+OLI | 3,4 | 0 |
| $H_2O$ | 4,8 | (ber.) 40,0 |
| MTBE | (ber.) 53,0 | 0,04 |
| $SO_4^{2-}$ | 0,2 | 47,7 |
| $NH_4^+$ | 0,02 | 9,7 |

Versuch 2

Versuch 1 wurde wiederholt mit 2,5 kg MHA-Hydrolysat und 1,5 kg MTBE, das bei der Eindampfung von MHA-MTBE-Extraktlösung (vgl. Beispiel 3) zurückgewonnen worden war. Die Zusammensetzungen in [Gew%] sind der nachfolgenden Tabelle 2 entnehmbar.

Tabelle 2

| | Organische Phase (2,6 kg) [Gew.-%] | Raffinat-Phase (1,4 kg) [Gew.-%] |
|---|---|---|
| MHAges | 42,0 | 1,9 |
| MHA | 37,7 | 1,9 |
| DIM+OLI | 4,3 | 0 |
| $H_2O$ | 4,6 | (ber.) 40,0 |
| MTBE | (ber.) 53 | 0,04 |
| $SO_4^{2-}$ | 0,2 | 47,7 |
| $NH_4^+$ | 0,015 | 9,7 |

Beispiel 3: Herstellung von MHA-Hochkonzentrat

In Figur 5 ist der schematische Aufbau der für Beispiel 3 verwendeten Apparatur gezeigt. Diese besteht im wesentlichen aus folgenden Apparaten:

| | |
|---|---|
| 001 | Vorratsbehälter |
| 002 } 003 } | Sambayverdampfer mit je 0,06 m² Austauschfläche und beheiztem Doppelmantel |
| 004 | Auffangbehälter für MHA-Produkt |
| 005 } 006 } | Kondensationssystem für abdestilliertes Lösungsmittel bestehend aus je einem wassergekühlten und einem solegekühlten Laborkühler, je einem Auffangbehälter und einer nachgeschalteten Wasserstrahlpumpe mit einstellbarem Vakuum |

Verfahrensbeschreibung unter Bezugnahme auf Figur 5:

Die aus der Extraktion kommende MHA-MTBE-Extraktlösung wird aus dem Vorratsbehälter 001 kontinuierlich in den Sambayverdampfer 002 eingespeist, der von außen beheizt wird. Der Ablauf aus 002 wird über ein Nadelventil in den ebenfalls beheizten Sambayverdampfer 003 eingespeist, dessen MHA-Produktablauf im Auffangbehälter 004 gesammelt und dort analysiert wird. Das v.a. aus Lösungsmittel bestehende Destillat wird in Auffangbehältern der beiden Kondensationssysteme 005 und 006 gesammelt und kann von dort zur Extraktion zurückgeführt werden (vgl. Beispiel 2, Versuch 2).

Versuch 3

Einsatz von 0,95 1/h (0,85 kg/h) MHA-MTBE-Extraktlösung aus Beispiel 2 Versuch 1

Sambay 002:

- Druck 250 mbar
- Temperaturen:
  - Heizmantel 125° C
  - Ablauf 79° C

Zusammensetzung des MHA-Hochkonzentrats im Sumpfablauf 002

MHAges: 98,0 Gew%
$H_2O$: 0,5 Gew%

Sambay 003:

- Druck 50 mbar
- Temperaturen:
  - Heizmantel 140° C
  - Ablauf 90° C
  - Brüden 30° C

Zusammensetzung des MHA-Hochkonzentrats im Sumpfablauf 003

| | | | |
|---|---|---|---|
| MHAges | 99,0 Gew% | MHA | 83,9 Mol% |
| | | DIM+OLI | 16,1 Mol% |
| $H_2O$ | 0,5 Gew% | | |
| MTBE | < 10 ppm | | |

Aus dem Sumpfablauf des Sambayverdampfers 003 wurden 0,36 kg/h MHA-Hochkonzentrat mit der obengenannten Zusammensetzung erhalten.

Versuch 4

Einsatz von 0,96 l/h (0,86 kg/h) MHA-MTBE-Extraktlösung aus Beispiel 2 Versuch 2

Sambay 002:

- Druck 250 mbar
- Temperaturen:
  - Heizmantel 125° C
  - Ablauf 96° C

Zusammensetzung des MHA-Hochkonzentrats im Sumpfablauf 002

| | |
|---|---|
| MHAges | 98,5 Gew% |
| $H_2O$ | 0,9 Gew% |

Sambay 003:

- Druck 50 mbar
- Temperaturen:
  - Heizmantel 120° C
  - Ablauf 100° C

- Brüden 28° C

Zusammensetzung des MHA-Hochkonzentrats im Sumpfablauf 003

| | | | |
|---|---|---|---|
| MHAges | 100,0 Gew% | MHA | 85,7 Mol% |
| | | DIM+OLI | 14,3 Mol% |
| $H_2O$ | 0,0 Gew% | | |
| MTBE | < 1 ppm | | |

Aus dem Sumpf ablauf des Sambayverdampfers 003 wurden 0,36 kg/h MHA-Hochkonzentrat mit der obengenannten Zusammensetzung erhalten.

Beispiel 4: Rückgewinnung von Salz aus MHA-Hydrolysat vor der Extraktion durch Flüssig/Flüssig- und Flüssig/Fest-Phasentrennung (vgl. Figur 1)

Versuch 5: MHA-Abtrennung mit Flüssig/Flüssig-Phasentrennung und Flüssig/Flüssig-Extraktion mit MTBE

Es wurden 502 g MHA-Hydrolysat mit 43,7 Gew% (219,4 g) MHAges (hergestellt wie unter Beispiel 1 beschrieben) bei einem Druck von 50 mbar auf einen Gehalt von 50 Gew.% MHAges eingedampft. Das Konzentrat (438,7 g) bestand aus 2 flüssigen Phasen, die bei T = 65° C voneinander getrennt wurden. Die Zusammensetzung der beiden Phasen ist in Tabelle 3 wiedergegeben:

Tabelle 3

| | organische Phase (292,4 g) | wäßrige Phase (143,4 g) |
|---|---|---|
| MHAges | 73,0 Gew% ≙ 213,4 g ≙ 97,3 % d.Th. | 3,9 Gew% ≙ 5,6 g ≙ 2,6 %d.Th. |
| $H_2O$ | 10,6 Gew% ≙ 31,0 g | 20,6 Gew% ≙ 29,5 g |
| Salz(ber.) | 16,4 Gew% ≙ 48,0 g | 75,5 Gew% ≙ 108,3 g |

Die wäßrige Phase wurde auf T = 26° C abgekühlt. Das dabei ausgefallene Salzkristallisat bestehend aus $NH_4HSO_4$ + $(NH_4)_2SO_4$ wurde abfiltriert. Es wurde die in Tabelle 4 angegebene Zusammensetzung erhalten:

Tabelle 4

| | Salzkristallisat (39,8 g) | wäßriges Filtrat (103,6 g) |
|---|---|---|
| MHAges | 0,1 Gew% (ber.) ≙ 0,42 g ≙ 0,2 % d.Th. | 5,0 Gew% ≙ 5,18 g ≙ 2,4 % d.Th. |
| $H_2O$ | 7,0 Gew% ≙ 2,8 g | 25,8 Gew% ≙ 26,7 g |
| Salz(ber.) | 92,9 Gew% ≙ 37,0 g | 69,2 Gew% ≙ 71,72 g |

Das Salzkristallisat wurde auf dem Filter mit 10 g MTBE gewaschen und das erhaltene organische Filtrat (6,0 g) analysiert (4,8 g Verdampfungsverlust MTBE) :
MHAges = 7,4 Gew% 0,44 g = 0,2 % d.Th.
MHA-Verlust über Salzkristallisat: < 0,2 % d.Th. ohne Salzwäsche
und 0 % d.Th. mit Salzwäsche.

Das gewaschene Salzkristallisat wurde getrocknet ( 35,8 g) und analysiert:

| | |
|---|---|
| $SO_4^{2-}$ | 80, 5 Gew% |
| $NH_4^+$ | 18,5 Gew% |
| Salz | 22,3 % d.Th. |

Die organische Phase (292,4 g), das wäßrige Filtrat (103,6 g) und das organische Filtrat (6,0 g) wurden mit 232 g MTBE gemischt und bei RT kurze Zeit intensiv gerührt. Nach Beendigung des Rührens wurden die beiden entstandenen Flüssigphasen voneinander abgetrennt. Die voneinander getrennten Phasen wiesen die Zusammensetzung wie in Tabelle 5 angegeben auf:

Tabelle 5:

| | organische Extraktlösung (475 g) | Raffinat (159 g) |
|---|---|---|
| MHAges | 46,0 Gew% ≙ 218,5 g (ber.) ≙ 98,4 % d. Th. | 1,8 Gew% ≙ 2,86 g ≙ 1,3 % d. Th. |
| $H_2O$ | 3,5 Gew% ≙ 16,6 g | 25,3 Gew% ≙ 40,2 g |
| $NH_4^+$ | 0,034 Gew% ≙ 0,2 g | n.b. |
| $SO_4$2- | 0, 55 Gew% ≙ 2,6 g | n.b. |
| Salz (ber.) | | 72,9 Gew% ≙ 115,9 g |

Der Restgehalt an MHAges aus dem Raffinat der hier beschriebenen einstufigen Extraktion läßt sich durch einbis mehrmaliges Nachextrahieren mit frischem Lösungsmittel bzw. durch kontinuierliches Extrahieren in einem System mit mehreren theoretischen Trennstufen auf < 0,1 % d.Th. verringern.

Das Salzkristallisat (35,8 g) wurde im Raffinat (159 g) bei 61° C klargelöst. Die so erhaltene Salzlösung hatte die folgende Zusammensetzung:

| | |
|---|---|
| MHAges | 1,4 Gew% ≙ 1,3 % d.Th. |
| $H_2O$ | 20,7 Gew% |
| Salz | 77,9 Gew%. |

Die so erhaltene Lösung kann besonders vorteilhaft einer Anlage zur Wiedergewinnung von Schwefelsäure zugeführt werden, da ihr Salzgehalt deutlich über 60 Gew% liegt.

Versuch 6: MHA-Abtrennung mit Flüssig/Flüssig-Phasentrennung und Flüssig/Flüssig-Extraktion mit MIBK

Es wurden 505 g MHA-Hydrolysat mit 43,7 Gew% (220,7 g) MHAges (hergestellt wie unter Beispiel 1 beschrieben) bei einem Druck von 50 mbar auf einen Gehalt von 49,9 Gew.% MHAges eingedampft. Das Konzentrat (440 g) bestand aus 2 flüssigen Phasen, die bei T = 60° C voneinander getrennt wurden. Die Zusammensetzung der Phasen wird in Tabelle 6 wiedergegeben:

Tabelle 6

| | organische Phase (299 g) | wäßrige Phase (141 g) |
|---|---|---|
| MHAges | 71,8 Gew% ≙ 214,7 g ≙ 97,3 % d. Th. | 4,0 Gew% ≙ 5,6 g ≙ 2,5 % d. Th. |

Die wäßrige Phase wurde auf T = 20° C abgekühlt. Das dabei ausgefallene Salzkristallisat bestehend aus $NH_4HSO_4$ + $(NH_4)_2SO_4$ wurde abfiltriert. Seine Zusammensetzung ist der Tabelle 7 entnehmbar.

Tabelle 7

| | Salzkristallisat (56 g) | wäßriges Filtrat (83 g) |
|---|---|---|
| MHAges | 2,5 Gew%(ber.) ≙ 1,4 g ≙ 0,6 % d.Th. | 5,0 Gew% ≙ 4,15 g ≙ 1,9 % d.Th. |

Das Salzkristallisat wurde auf dem Filter mit 14 g MIBK gewaschen und das erhaltene organische Filtrat (13,8 g) analysiert.
MHAges = 9 Gew% ≙ 1,2 g ≙ 0,56 % d.Th.
MHA-Verlust über Salzkristallisat < 0,1 % d.Th.

Das gewaschene Salzkristallisat wurde getrocknet ( 40 g) und analysiert:

| | |
|---|---|
| $SO_4^{2-}$ | 80,7 Gew% |
| $NH_4^+$ | 18,8 Gew% |
| Salz | 24,7 % d.Th. |

Die organische Phase (299 g), das wäßrige Filtrat (83 g) und das organische Filtrat (13.8 g) wurden mit 250 g MIBK gemischt und bei RT kurze Zeit intensiv gerührt. Nach Beendigung des Rührens wurden die beiden entstandenen Flüssigphasen voneinander abgetrennt. Diese wiesen die in Tabelle 8 angegebenen Zusammensetzung auf.

Tabelle 8

| | org. Extraktlösung (484 g) | Raffinat (144 g) |
|---|---|---|
| MHAges | 217,1 g (ber.) ≙ 98,4 % d. Th. | 2,05 Gew% ≙ 2,95 g ≙ 1,3 % d. Th. |

Der Restgehalt an MHAges aus dem Raffinat der hier beschriebenen einstufigen Extraktion läßt sich durch einbis mehrmaliges Nachextrahieren mit frischem Lösungsmittel bzw. durch kontinuierliches Extrahieren in einem System mit mehreren theoretischen Trennstufen auf < 0,1 % d.Th. verringern.

Beispiel 5: Rückgewinnung von Salz aus MHA-Hydrolysat vor der Extraktion durch Flüssig/Fest-Phasentrennung (vgl. Figur 2)

Versuch 7: MHA-Abtrennung ohne Flüssig/Flüssig-Phasentrennung

Es wurden 505 g MHA-Hydrolysat mit 43,7 Gew% (220,7 g) MHAges (hergestellt wie unter Beispiel 1 beschrieben) bei einem Druck von 50 mbar auf einen Gehalt von 49,9 Gew% MHAges eingedampft. Das Konzentrat (440 g) wurde auf RT abgekühlt, wobei eine Suspension aus Salzkristallisat und einer homogenen Flüssigphase erhalten wurde, welche durch Filtration getrennt wurde. Die in Tabelle 9 gezeigte Zusammensetzung wurde gefunden.

Tabelle 9

| | Filtrat (342,4 g) | Salzkristallisat (95,0 g) |
|---|---|---|
| MHAges | 56,6 Gew% ≙ 193,8 g ≙ 87,8 % d.Th. | 34,4 Gew%(ber.) ≙ 32,7 g ≙ 14,8 % d. Th. |

Das Salzkristallisat wurde auf dem Filter mit 20 g MIBK gewaschen und das organische Filtrat (41,6 g) analysiert: MHAges: 52,5 Gew% ≙ 21,8 g = 9,9 % d.Th.

Das gewaschene Salzkristallisat wurde getrocknet (52,8 g) und analysiert:

| | |
|---|---|
| MHAges | 6,1 Gew% = 1,5 % d.Th. |
| $SO_4^{2-}$ | 75,0 Gew% |
| $NH_4^+$ | 18,4 Gew% |
| Salz | 93,9 Gew% = 30,7 d.Th. |

Der MHA-Verlust über das gewaschene Salzkristallisat betrug 1,5 % d.Th.

Das Filtrat (342,4 g) wurde bei RT in 244 g MIBK aufgenommen und mit dem organischen Filtrat (41,6 g) versetzt, wobei sich eine wäßrige Flüssigphase absetzte. Beide Flüssigphasen wurden voneinander getrennt und es wurde die in der Tabelle 10 gezeigte Zusammensetzung gefunden.

Tabelle 10

| | org. Extraktlösung (480 g) | Raffinat (140 g) |
|---|---|---|
| MHAges | 212,5 g (ber.) ≙ 96,3 % d.Th. | 2,25 Gew% ≙ 3,15 g ≙ = 1,4 % d. Th. |

Der Restgehalt an MHAges aus dem Raffinat der hier beschriebenen einstufigen Extraktion läßt sich durch ein- bzw. mehrmaliges Nachextrahieren mit frischem Lösungsmittel bzw. durch kontinuierliches Extrahieren in einem System mit mehreren theoretischen Trennstufen auf < 0,1 % d.Th. verringern.

Der Restgehalt an MHAges im Salzkristallisat kann durch weiteres Nachwaschen mit Lösungsmittel oder Wasser weiter abgesenkt werden. Ein Nachwaschen mit Wasser geschieht bevorzugt mit einer wäßrigen $NH_4HSO_4$- und/oder $(NH_4)_2SO_4$-Lösung, die wiederum bevorzugt mehrmals eingesetzt und spätestens bei Erreichen ihrer vollständigen Beladung in das Extraktionssystem zurückgeführt wird, zwecks Rückextraktion von darin gelöstem MHAges.

Das MHA-haltige organische Filtrat kann direkt dem Lösungsmittelextraktionssystem zur MHA-Isolierung aus der organischen und/oder aus dem wäßrigen Filtrat zugeführt werden. Dabei wird vorteilhafterweise ein MHA-Verlust von ca. 0,5 - 12,5 % d.Th. vermieden.

Die Salzkristallisate aus den Beispielen 4 und 5 sind geeignet zur Umarbeitung in verkaufsfähiges $(NH_4)_2SO_4$, durch Zugabe von entsprechenden Anteilen $NH_3$ und anschließender Kristallisation. Ebenso können sie direkt oder

vorzugsweise nach Auflösung in Wasser, oder in einer geeigneten $NH_4HSO_4$ bzw. $(NH_4)_2SO_4$, oder beide Salze enthaltende Lösung einer Anlage zur $H_2SO_4$-Rückgewinnung zugeführt werden.

Beispiel 6: Flüssig/Flüssig-Extraktion von MHA-Hydrolysat (vgl. Figur 3)

Versuch 8: Extraktion mit MTBE

Es wurden 100 g MHA-Hydrolysat mit 43,7 Gew% (43,7 g) MHAges (hergestellt wie unter Beispiel 1 beschrieben) bei RT mit 60 g MTBE gemischt und kurze Zeit intensiv gerührt. Nach Beendigung des Rührens wurden die beiden entstandenen Flüssigphasen voneinander getrennt. Die Ergebnisse sind in Tabelle 11 zusammengestellt:

Tabelle 11

| | org. Extraktlösung (107 g) | Raffinat (52 g) |
|---|---|---|
| MHAges | 39,9 Gew% (43,6 g) = 97,7 % d.Th. | 2,0 Gew% (1,0 g) = 2,3 % d.Th. |
| $H_2O$ | 4,16 Gew% | 38,7 Gew% |
| $NH_4^+$ | 0,02 Gew% | 9,6 Gew% |
| $SO_4^{2-}$ | 0,21 Gew% | 50,3 Gew% |

Versuch 9: Extraktion mit MIBK

Es wurden 100 g MHA-Hydrolysat mit 43,7 Gew% MHAges analog Versuch 8 mit 60 g MIBK extrahiert (Tabelle 12) :

Tabelle 12

| | org. Extraktlösung (107,5 g) | Raffinat (51,5 g) |
|---|---|---|
| MHAges | 39,0 Gew% (41,9 g) = 95,9 % d.Th. | 2,4 Gew% (1,2 g) = 2,8 % d.Th. |
| $H_2O$ | 4,65 Gew% | 38,2 Gew% |
| $NH_4^+$ | 0,05 Gew% | 9,7 Gew% |
| $SO_4^{2-}$ | 0,38 Gew% | 50,4 Gew% |

Wie der Vergleich der beiden einstufigen Lösungsmittelextraktionen zeigt, nimmt die organische Extraktlösung im Falle des MTBE (Versuch 8) nur etwa halb so viel des unerwünschten anorganischen Ammoniumsalzes auf, wie im Falle des MIBK (Versuch 9). Bei Verwendung von MTBE wird außerdem noch weniger MHA über die Raffinatphase verloren.

Beispiel 7: MHA-Isolierung nach Aufstockung des Salzgehalts (vgl. Figur 4)

Versuch 10: MHA-Abtrennung mit Flüssig/Flüssig-Phasentrennung und Flüssig/Flüssig-Extraktion

Es wurden 23 g $(NH_4)_2SO_4$ in 598 g MHA-Hydrolysat mit 43,7 Gew% (261,3 g) MHAges (hergestellt wie unter Beispiel 1 beschrieben) bei T = 60° C aufgelöst. Die Lösung (621 g) bestand aus 2 flüssigen Phasen, die bei T = 60° C getrennt wurden. Folgende Zusammensetzung (Tabelle 13) wurde gefunden:

Tabelle 13

| | organische Phase (416 g) | wäßrige Phase (205 g) |
|---|---|---|
| MHAges | 58,7 Gew% ≙ 244,2 g ≙ 93,4 % d. Th. | 7,8 Gew% ≙ 16 g ≙ 6,1 % d. Th. |
| $H_2O$ | 20,4 Gew% ≙ 84,9 g | 32,7 Gew% ≙ 67,0 g |
| Salz (ber.) | 20,9 Gew% ≙ 86,9 g | 59,5 Gew% ≙ 122,0 g |

Die wäßrige Phase mit einem Salzgehalt von ca. 60 Gew.% kann direkt einer Anlage zur Wiedergewinnung von Schwefelsäure zugeführt werden.

Die organische Phase (416 g) wurde mit 250 g MTBE gemischt und bei RT kurze Zeit intensiv gerührt. Nach Beendigung des Rührens wurden die beiden entstandenen Flüssigphasen voneinander abgetrennt. Deren Zusammensetzung zeigt Tabelle 14.

Tabelle 14

| | org. Extraktlösung (522 g) | Raffinat (144 g) |
|---|---|---|
| MHAges | 47,5 Gew% ≙ 248,0 g (ber.) ≙ 94,9 % d. Th. | 2,4 Gew% ≙ 3,4 g ≙ 1,3 % d. Th. |
| $H_2O$ | 5,5 Gew% ≙ 28,8 g | 39,0 Gew% ≙ 56,2 g |
| $NH_4^+$ | 0,032 Gew% ≙ 0,2 g | n.b. |
| $SO_4^{2-}$ | 0,28 Gew% ≙ 1,5 g | n.b. |
| Salz (ber.) | - - - | 58,6 Gew% ≙ 84,4 g |

Der Restgehalt an MHAges aus dem Raffinat der hier beschriebenen einstufigen Extraktion läßt sich durch einbis mehrmaliges Nachextrahieren mit frischem Lösungsmittel bzw. durch kontinuierliches Extrahieren in einem System mit mehreren theoretischen Trennstufen auf < 0,1 % d.Th. verringern.

Die wäßrige Phase (205 g) und das Raffinat (144 g) wurden vereinigt. Die so erzeugte Salzlösung (349 g) hatte die folgende Zusammensetzung:

| | |
|---|---|
| MHAges | 5,6 Gew% |
| $H_2O$ | 35,3 Gew% |
| Salz | 59,1 Gew%. |

Die ca. 60%ige Salzlösung kann direkt einer Anlage zur Wiedergewinnung von Schwefelsäure zugeführt werden. Eine weitere Aufkonzentration kann durch vergrößerten Salzeinsatz im Hydrolysat und durch kontinuierliches Extrahieren und vollständige MHA-Abtrennung aus dem Raffinat erreicht werden.

Versuch 11: MHA-Abtrennung mit Flüssig/Flüssig-Extraktion

Analog zu Versuch 10 wurden 23 g $(NH_4)_2SO_4$ in 598 g MHA-Hydrolysat gelöst. Das entstandene Gemisch aus 2 Flüssigphasen (620 g) wurde mit 372 g MTBE versetzt und bei T = 40°C intensiv gerührt. Nach Beendigung des Rührens wurden die beiden entstandenen Flüssigphasen voneinander abgetrennt. Die Ergebnisse faßt Tabelle 15 zusammen.

Tabelle 15

| | org. Extraktlösung (658 g) | Raffinat (332 g) |
|---|---|---|
| MHAges | 38,8 Gew% ≙ 255,0 g (ber.) ≙ 97,6 % d. Th. | 1,9 Gew% ≙ 6,3 g ≙ 2,4 % d. Th. |
| $H_2O$ $H_2O$ | 4,4 Gew% ≙ 30,0 g | 37,0 Gew% ≙ 122,8 g |
| $NH_4^+$ | 0,012 Gew% ≙ 0,08 g | n.b. |
| $SO_4^{2-}$ | 0,15 Gew% ≙ 0,99 g | n.b. |
| Salz (ber.) | | 61,1 Gew.% ≙ 202,9 g |

Der Restgehalt an MHAges aus dem Raffinat der hier beschriebenen einstufigen Extraktion läßt sich durch einbis mehrmaliges Nachextrahieren mit frischem Lösungsmittel bzw. durch kontinuierliches Extrahieren in einem System mit mehreren theoretischen Trennstufen auf < 0,1 % d.Th. verringern.

Das Raffinat mit einem Salzgehalt von > 60 Gew% kann direkt einer Anlage zur Wiedergewinnung von Schwefelsäure zugeführt werden.

Sowohl bei Versuch 10 als auch bei Versuch 11 kann auf ein Waschen der organischen Extraktlösung mit Wasser verzichtet werden, da der restliche Sulfatsalzgehalt bereits extrem niedrig liegt. Das stellt einen großen Vorteil dar, da sowohl verfahrenstechnischer Zusatzaufwand als auch die unerwünschte Raffinatverdünnung auf diese Weise vermieden werden kann.

Die in Beispiel 4, 5, 6 bzw. 7 erzeugten organischen Extraktlösungen können analog Beispiel 2 bzw. 8 kontinuierlich eingedampft werden bis zur praktisch vollständigen Lösungsmittelfreiheit und auf einen Wassergehalt von

< 5 Gew%. Das so erzeugte MHA-Hochkonzentrat kann durch entsprechende Konditionierung in verschiedene MHA-Produktmischungen überführt werden.

Beispiel 8: Verfahrensbeschreibung unter Bezugnahme auf Figur 6

In Figur 6 ist der schematische Aufbau der für Beispiel 8 verwendeten Apparatur gezeigt. Die verwendeten Bezugszeichen bezeichnen folgende Apparate aus welchen die verwendete Apparatur im wesentlichen besteht:

| | |
|---|---|
| (001) | Extraktionskolonne, z.B. eine pulsierte Siebbodenkolonne mit 3 m Länge, 2,1 cm Innendurchmesser, 60 Siebböden, beheiztem Doppelmantel; |
| (002) | Dünnschichtverdampfer, z.B. ein Sambay-Verdampfer mit 0,08 $m^2$ Austauschfläche, beheiztem Doppelmantel; |
| (003) | Kondensationssystem, z.B. ein wassergekühlter Glaskühler; |
| (004/005) | Auffangbehälter für zurückgeführtes Wasser bzw. Lösungsmittel |
| (006) | Phasentrenner für organisches und wäßriges Raffinat |
| (007) | Waschzone für überlaufende Extraktlösung |

Das aus der MHA-Hydrolysestufe kommende MHA-Hydrolysat, das im wesentlichen besteht aus MHA (Monomer + Dimere + Oligomere + ggf. Amid), $(NH_4)_2SO_4$ und/oder $NH_4HSO_4$ sowie Wasser, wird nach Vorheizung auf die Extraktionstemperatur oberhalb des 40sten Bodens in die Extraktionskolonne 001 eindosiert. Das Lösungsmittel (hier Methylisobutylketon = MIBK) wird ebenfalls vorgeheizt in den Sumpf der Kolonne gepumpt (Gegenstromprinzip). Zusätzlich wird der Überlauf der Kolonne in einer Waschzone mit Waschwasser beaufschlagt und die Waschphase in den Hydrolysateingangsstrom zurückgeführt. Das im wesentlichen $(NH_4)_2SO_4$ und/oder $NH_4HSO_4$ sowie Wasser enthaltende wäßrige Raffinat sowie das hauptsächlich aus Lösungsmittel bestehende organische Raffinat wird unter Kühlung gemeinsam am Kolonnensumpf abgezogen. Die beiden Phasen werden in einem Phasentrenner 006 getrennt, das organische Raffinat dem Extraktionssystem zurückgeführt, das wäßrige Raffinat ausgeschleust. Die im wesentlichen MHA, Lösungsmittel sowie Wasser enthaltende Extraktionslösung wird am Kolonnenkopf abgeführt und anschließend nach Passieren der Waschzone 007 in den Sambay-Verdampfer 002 eingespeist. Dort wird unter Anlegen eines Vakuums und unter zusätzlichem Einblasen von $H_2O$-Dampf sowie eines $N_2$-Stromes kurz vor dem Ablauf des Verdampfers MIBK und $H_2O$ gemeinsam aus der Extraktionslösung entfernt. Die Verdampfung wurde so gefahren, daß im Sambayablauf < 2 Gew% $H_2O$ nachweisbar waren und das ablaufende MHA-Hochkonzentrat praktisch lösungsmittelfrei war.

Das aus dem Verdampfer 002 kommende Lösungsmittel/Wassergemisch wurde zunächst in 003 kondensiert und zur Trennung in ein Separationsgefäß geführt. Wasser bzw. Lösungsmittel wurden jeweils in einem Auffangbehälter 004 bzw. 005 gesammelt und von da aus in das Extraktionssystem zurückgeführt. Der Sambayablauf wurde auf Raumtemperatur gekühlt in einen Produktauffangbehälter geführt.

Die Zusammensetzung der Extraktionslösung wurde unmittelbar nach der Waschzone 007, die Zusammensetzung der wäßrigen bzw. der organischen Raffinatlösung jeweils unmittelbar nach dem Phasentrenner 006 analysiert.

Die Zusammensetzung des MHA-Hochkonzentrats wurde im Sambay-Sumpfablauf unmittelbar nach der Austrittsstelle ermittelt.

Die für die Extraktion eingesetzte MHA-Hydrolysat-Lösung wurde in einem druckstabilen 400-1-Rührkessel aus 114,7 kg (874 mol) MMP-Cyanhydrin und 131,9 kg (874 mol, 1,00 moleq) 65 % $H_2SO_4$ bei einer Temperatur von 50 °C und 60 min Verweilzeit, anschließender Verdünnung mit 96,7 kg $H_2O$ und Weiterreaktion bei einer Temperatur von 90 °C und 120 min Verweilzeit hergestellt. Die Roh-Hydrolysatlösung wurde nach Beendigung der Reaktion durch Anlegen eines Vakuums von vorhandenen leichtflüchtigen Nebenprodukten befreit und anschließend analysiert. Die dabei erhaltene Zusammensetzung des zur Extraktion eingesetzten MHA-Hydrolysats ist bei Versuch 12 zu Beispiel 8 angegeben.

Die Bedingungen und Ergebnisse des Versuchs 12 sind im folgenden tabellarisch zusammengefaßt.

Versuch 12: Einsatz von MHA-Hydrolysat aus MMP-Cyanhydrin und 1,0 moleq $H_2SO_4$

Einsatz in Extraktion:

| | | |
|---|---|---|
| Mengenströme | - MIBK<br>- MHA-Hydrolysat<br>- MHA-Gesamt<br>- Wasch-$H_2O$<br>- MIBK/Hydrolysat | 6,7 kg/h<br>12,3 kg/h<br>4,9 kg/h<br>1,3 kg/h<br>0,55 [-] |

Zusammensetzung des MHA-Hydrolysats:

| | |
|---|---|
| - MHAges | 39,5 Gew.% |
| - MHA | 94,7 Mol% |
| - DIM+OLI | 5,3 Mol% |
| - $H_2O$ | 28,7 Gew.% |
| - $SO_4$2- | 27,5 Gew.% |
| - ($NH_4HSO_4$) | 33,2 Gew.% |

Extraktion (001):

| | |
|---|---|
| Temperatur | 60° C (Durchschnitt) |

Zusammensetzungen

| | | |
|---|---|---|
| - der Extraktionslösung: | MIBK<br>MHAges<br>$H_2O$ | 44,7 Gew.% (ber.)<br>41,8 Gew.%<br>13,5 Gew.% |
| - des wäßrigen Raffinats | MIBK<br>MHAges | 77,5 ppm<br>0,1 Gew.% |
| - des organischen Raffinats (Mengenstrom 0,017 kg/h) | MIBK | 97,5 Gew.% |

Eindampfung 002

| | | |
|---|---|---|
| Druck | | 600 mbar |
| Sambay | - Temperatur | |
| | im Heizmantel | 180° C |
| | im Kopf | 85° C |
| | im Sumpf | n.b. |
| | - Strippdampf | 0,5 kg/h |
| | - Strippgas $N_2$ | 100 l/h |

Zusammensetzung des MHA-Hochkonzentrats im Sumpfablauf:

| | |
|---|---|
| MHAges | 98 Gew.% |
| MHA | 86 Mol% |
| DIM+OLI | 14 Mol% |
| $H_2O$ | 2 Gew.% |

(fortgesetzt)

| | |
|---|---|
| MIBK | 40 ppm |

Aus dem Sambay-Sumpfablauf wurden ca. 4,9 kg/h MHA-Hochkonzentrat mit der oben genannten Zusammensetzung erhalten. Das organische Raffinat wurde in die Extraktionskolonne zurückgeführt. Das wäßrige Raffinat wurde direkt und ohne weitere Nachbehandlung zur Entsorgung ausgeschleust.

Eine weitere Destillations- bzw. Strippstufe zur Entfernung von Restlösungsmittel aus dem Sumpfablauf der Kolonne konnte so umgangen werden. Das organische Raffinat, das unter milden Bedingungen als dritte Flüssigphase aus der Extraktionskolonne abgezogen wurde, konnte zudem ohne weitere Aufreinigung dieser direkt wieder zugeführt werden.

**Patentansprüche**

1. Verfahren zur Gewinnung von 2-Hydroxy-4-methylthiobuttersäure (MHA), bei dem das MHA aus einem Reaktionsgemisch isoliert wird, welches durch Anlagerung von Blausäure (HCN) an Methylmercaptopropionaldehyd (MMP) und Hydrolyse des dabei erhaltenen Methylmercaptopropionaldehyd-Cyanhydrins (MMP-CH) mit Schwefelsäure erhalten wird, wobei das Reaktionsgemisch mit einem im wesentlichen mit Wasser nicht mischbaren organischen Lösungsmittel in einem Flüssig/Flüssig-Extraktionssystem in Kontakt gebracht wird, um eine Extraktionslösung zu bilden, die das Lösungsmittel und aus dem Reaktionsgemisch überführtes MHA aufweist, und das MHA als Extrakt aus dieser Extraktionslösung durch Eindampfen gewonnen wird,
**dadurch gekennzeichnet,**
daß der Salzgehalt des Reaktionsgemisches vor der Flüssig/Flüssig-Extraktion auf eine Konzentration von > 50 Gew.-% (wt/wt), bevorzugt > 55 Gew.-% (wt/wt), bezogen auf die Summe der nicht organischen Bestandteile des Reaktionsgemisches, gebracht und die Hydrolyse des MMP-CH so geführt wird, daß in einer ersten Stufe das MMP-CH mit 60 bis 85 %iger Schwefelsäure im Molverhältnis MMP-CH: $H_2SO_4$ von 1 : 0,5 bis 1 : 1,0 bei Temperaturen von 30 bis 90°C unter Erhalt von im wesentlichen MHA-Amid hydrolysiert und dieses in einer zweiten Stufe unter Zugabe von Wasser ohne weitere Zugabe von $H_2SO_4$ bei Temperaturen bis 140°C hydrolysiert wird.

2. Verfahren nach Anspruch 1,
**dadurch gekennzeichnet,**
daß dem durch Anlagerung von HCN an MMP und Hydrolyse des entstandenen MMP-CH mit Schwefelsäure entstandenen Reaktionsgemisch Ammoniumsulfat zugesetzt wird.

3. Verfahren nach Anspruch 2,
**dadurch gekennzeichnet,**
daß vor Isolierung des MHA eine zum Aussalzen wirksame Menge Ammoniumsulfat zugefügt wird.

4. Verfahren nach Anspruch 1,
**dadurch gekennzeichnet,**
daß die Salzkonzentration durch Eindampfung angehoben wird.

5. Verfahren nach einem oder mehreren der vorhergehenden Ansprüche, **dadurch gekennzeichnet,**
daß unmittelbar aus dem Extraktionssystem wenigstens drei flüssige Phasen resultieren.

6. Verfahren nach Anspruch 5, **dadurch gekennzeichnet,**
daß ein homogener Extrakt und ein aus zwei flüssigen Phasen bestehendes Raffinat gebildet wird.

7. Verfahren nach Anspruch 6,
**dadurch gekennzeichnet,**
daß das Raffinat als erste flüssige Phase im wesentlichen aus Ammoniumsalz und Wasser und zu geringen Teilen aus MHA und organischem Lösungsmittel besteht, während es als zweite flüssige Phase im wesentlichen aus organischem Lösungsmittel und zu geringen Teilen aus Wasser und MHA besteht.

8. Verfahren nach Anspruch 7, **dadurch gekennzeichnet,**
daß die erste flüssige Phase Wasser in einer Menge von 20 bis 50 Gew.-% enthält, MHA in einer Menge von 0,01 bis 0,5 Gew.-% enthält und Salz in einer Menge von 50 bis 80 Gew.-% enthält, während die zweite flüssige Phase

MHA in einer Menge von 0,01 bis 0,5 Gew.-% enthält, Lösungsmittel in einer Menge von 90 bis 99,9 Gew.-% enthält und Wasser in einer Menge von 0,1 bis 10 Gew.-% enthält, wobei die Bestandteile jeder Phase für sich genommen 100 Gew.-% ergeben.

**9.** Verfahren nach Anspruch 1,
**dadurch gekennzeichnet,**
daß im Molverhältnis MMP-CH : $H_2SO_4$ von 1 : 0,6 bis 1 : 0,95 hydrolysiert wird.

**10.** Verfahren nach einem oder mehreren der vorhergehenden Ansprüche,
**dadurch gekennzeichnet,**
daß als Lösungsmittel ein oder mehrere Ether zugesetzt werden.

**11.** Verfahren nach Anspruch 10,
**dadurch gekennzeichnet,**
daß ein asymmetrischer Ether zugesetzt wird.

**12.** Verfahren nach Anspruch 11,
d**adurch gekennzeichnet,**
daß ein Ether mit einem Siedepunkt < 60 °C zugesetzt wird.

**13.** Verfahren nach einem der Ansprüche 10 bis 12,
**dadurch gekennzeichnet,**
daß Methyltertiärbutylether (MTBE) zugesetzt wird.

**14.** Verfahren nach einem oder mehreren der vorhergehenden 5 Ansprüche,
**dadurch gekennzeichnet,**
daß die Extraktionslösung auf Wassergehalte < 4 % eingedampft wird und das restliche Lösungsmittel aus dem resultierenden Konzentrat abdestilliert wird.

**15.** Verfahren nach Anspruch 14,
**dadurch gekennzeichnet,**
daß mit Wasserdampf gestrippt wird.

**16.** Verfahren nach Anspruch 14 oder 15,
**dadurch gekennzeichnet,**
daß nach der Lösungsmittel-Entfernung ein Produkt mit Wassergehalt < 4 % resultiert.

**17.** Verfahren nach Anspruch 16,
**dadurch gekennzeichnet,**
daß ein Produkt mit Wassergehalt < 3 % resultiert.

## Claims

**1.** Process for the recovery of 2-hydroxy-4-methylthiobutyric acid (MHA), wherein the MHA is isolated from a reaction mixture which is obtained by addition of hydrocyanic acid (HCN) to methylmercaptopropionaldehyde (MMP) and hydrolysis with sulfuric acid of the methylmercaptopropionaldehyde cyanohydrin (MMP-CH) thus obtained,
the reaction mixture being brought into contact, in a liquid/liquid extraction system, with an organic solvent which is substantially immiscible with water, in order to form an extracting solution which contains the solvent and MHA transferred out of the reaction mixture, and the MHA being recovered as extract from this extracting solution by evaporation,
characterised in that
the salt content of the reaction mixture, prior to the liquid/liquid extraction, is brought to a concentration of > 50 wt. % (wt/wt), preferably > 55 wt.% (wt/wt), based on the sum of the inorganic constituents of the reaction mixture and the hydrolysis of the MMP-CH is carried out in such a way that, in a first step, the MMP-CH is hydrolysed with 60% to 85% sulfuric acid in the molar ratio MMP-CH:$H_2SO_4$ of 1:0.5 to 1:1.0 at temperatures of from 30°C to 90°C, with substantially MHA amide being obtained and this, in a second stage, is hydrolysed by addition of water at temperatures of up to 140°C without further addition of $H_2SO_4$.

**2.** Process according to claim 1, characterised in that ammonium sulfate is added to the reaction mixture formed by addition of HCN to MMP and hydrolysis with sulfuric acid of the MMP-CH formed.

**3.** Process according to claim 2, characterised in that prior to isolation of the MHA, a quantity of ammonium salt sufficient for the salting out is added.

**4.** Process according to claim 1, characterised in that the salt concentration is increased by evaporation.

**5.** Process according to one or more of the preceding claims, characterised in that at least three liquid phases result directly from the extraction system.

**6.** Process according to claim 5, characterised in that a homogeneous extract and a raffinate consisting of two liquid phases are formed.

**7.** Process according to claim 6, characterised in that the first liquid phase of the raffinate consists substantially of ammonium salt and water and small proportions of MHA and of organic solvent, whereas the second phase consists substantially of organic solvent and small proportions of water and of MHA.

**8.** Process according to claim 7, characterised in that the first liquid phase contains water in a quantity of 20 to 50 wt.%, contains MHA in a quantity of 0.01 to 0.5 wt.% and contains salt in a quantity of 50 to 80 wt.%, whereas the second liquid phase contains MHA in a quantity of 0.01 to 0.5 wt.%, contains solvent in a quantity of 90 to 99.9 wt.% and contains water in a quantity of 0.1 to 10 wt. %, the constituents of each phase on its own amounting to 100 wt.%.

**9.** Process according to claim 1, characterised in that the hydrolysis is carried out in the molar ratio MMP-CH:$H_2SO_4$ of 0.1:0.6 to 1:0.95.

**10.** Process according to one or more of the preceding claims, characterised in that one or more ethers are added as solvent.

**11.** Process according to claim 10, characterised in that an asymmetric ether is added.

**12.** Process according to claim 11, characterised in that an ether having a boiling point of < 60°C is added.

**13.** Process according to one of claims 10 to 12, characterised in that methyl tertiary butyl ether (MTBE) is added.

**14.** Process according to one or more of the preceding claims, characterised in that the extracting solution is evaporated to water contents of < 4% and the remaining solvent is distilled out of the resulting concentrate.

**15.** Process according to claim 14,
characterised in that
stripping is carried out using steam.

**16.** Process according to claim 14 or 15,
characterised in that
a product having a water content of < 4% is obtained after removal of the solvent.

**17.** Process according to claim 16
characterised in that
a product having a water content of < 3% is obtained.

**Revendications**

**1.** Procédé pour l'obtention d'acide 2-hydroxy-4-méthylthiobutyrique (MHA), dans lequel le MHA est isolé à partir d'un mélange réactionnel obtenu par addition d'acide cyanhydrique (HCN) sur du méthylmercaptopropionaldéhyde (MMP) et par hydrolyse de la méthylmercaptopropionaldéhyde-cyanhydrine (MMP-CN) ainsi obtenue avec de l'acide sulfurique, le mélange réactionnel étant mis en contact avec un solvant organique essentiellement non miscible avec de l'eau dans un système d'extraction liquide/liquide, pour former une solution d'extraction, qui présente le solvant et le MHA transféré à partir du mélange réactionnel, et le MHA étant obtenu comme extrait à partir de cette solution d'extraction, caractérisé en ce que la teneur en sel du mélange réactionnel avant l'extraction liquide/liquide est amenée à une concentration > à 50 % en poids (poids/poids), de préférence > à 55 % en poids (poids/poids), par rapport à la somme des constituants non organiques du mélange réactionnel, et en ce que l'hydrolyse du MMP-CH est conduite de telle manière qu'on hydrolyse, dans une première étape, le MMP-CH avec de l'acide sulfurique à 60 jusqu'à 85 % dans un rapport molaire MMP-CH: $H_2SO_4$ de 1:0,5 à 1:1,0 à des températures de 30 à 90 °C, en obtenant essentiellement du MHA-amide et en hydrolysant celui-ci dans une deuxième étape avec addition d'eau sans autre addition de $H_2SO_4$ à des températures jusqu'à 140 °C.

**2.** Procédé selon la revendication 1, caractérisé en ce qu'on ajoute du sulfate d'ammonium au mélange réactionnel obtenu par addition de HCN sur du MMP et par hydrolyse de la MMP-CN obtenue avec de l'acide sulfurique.

**3.** Procédé selon la revendication 2, caractérisé en ce qu'on ajoute une quantité de sulfate d'ammonium efficace pour le relargage avant l'isolement du MHA.

**4.** Procédé selon la revendication 1, caractérisé en ce que la concentration en sel est augmentée par concentration par évaporation.

**5.** Procédé selon l'une ou plusieurs des revendications précédentes, caractérisé en ce qu'on obtient directement à partir du système d'extraction au moins trois phases liquides.

**6.** Procédé selon la revendication 5, caractérisé en ce qu'un extrait homogène et un raffinat constitué par deux phases liquides sont formés.

**7.** Procédé selon la revendication 6, caractérisé en ce que le raffinat est constitué, comme première phase liquide, essentiellement de sel d'ammonium et d'eau et de faibles parties de MHA et de solvant organique, alors qu'il est constitué, comme deuxième phase liquide, essentiellement de solvant organique et de faibles parties d'eau et de MHA.

**8.** Procédé selon la revendication 7, caractérisé en ce que la première phase liquide contient de l'eau en une quantité de 20 à 50 % en poids, du MHA en une quantité de 0,01 à 0,5 % en poids et du sel en une quantité de 50 à 80 % en poids, alors que la deuxième phase liquide contient du MHA en une quantité de 0,01 à 0,5 % en poids, du solvant en une quantité de 90 à 99,9 % en poids et de l'eau en une quantité de 0,1 à 10 % en poids, les constituants de chaque phase prise séparément formant 100 % en poids.

**9.** Procédé selon la revendication 1, caractérisé en ce qu'on hydrolyse à un rapport molaire MMP-CH : $H_2SO_4$ de 1: 0,6 à 1:0,95.

**10.** Procédé selon l'une ou plusieurs des revendications précédentes, caractérisé en ce qu'on ajoute, comme solvant, un ou plusieurs éthers.

**11.** Procédé selon la revendication 10, caractérisé en ce qu'on ajoute un éther asymétrique.

**12.** Procédé selon la revendication 11, caractérisé en ce qu'on ajoute un éther présentant un point d'ébullition < à 60 °C.

**13.** Procédé selon l'une quelconque des revendications 10 à 12, caractérisé en ce qu'on ajoute du méthyltert.-butyléther (MTBE).

**14.** Procédé selon l'une ou plusieurs des revendications précédentes, caractérisé en ce que la solution d'extraction est concentrée par évaporation à une teneur en eau < à 4 % et en ce que le solvant résiduel est éliminé par distillation du concentrat qui en résulte.

**15.** Procédé selon la revendication 14, caractérisé en ce qu'on lave avec de la vapeur d'eau.

**16.** Procédé selon la revendication 14 ou 15, caractérisé en ce qu'on obtient après l'élimination du solvant un produit présentant une teneur en eau < à 4 %.

**17.** Procédé selon la revendication 16, caractérisé en ce qu'on obtient un produit présentant une teneur en eau < à 3 %.

Fig. 1: MHA-Isolierung nach Salzabtrennung und Flüssig/Flüssig-Phasentrennung im MHA-Hydrolysat

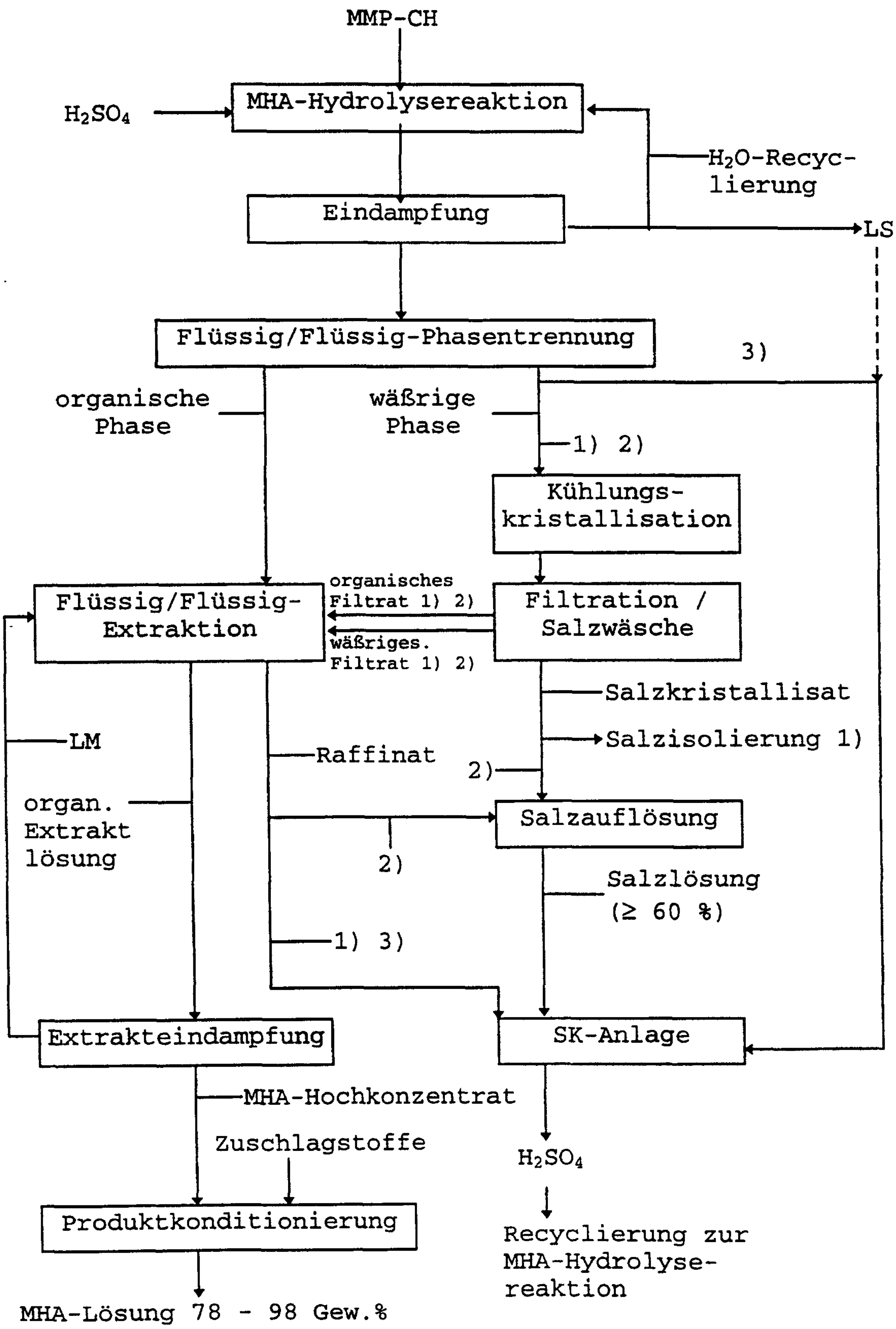

Fig. 2: MHA-Isolierung nach Salzabtrennung ohne Flüssig/Flüssigphasentrennung

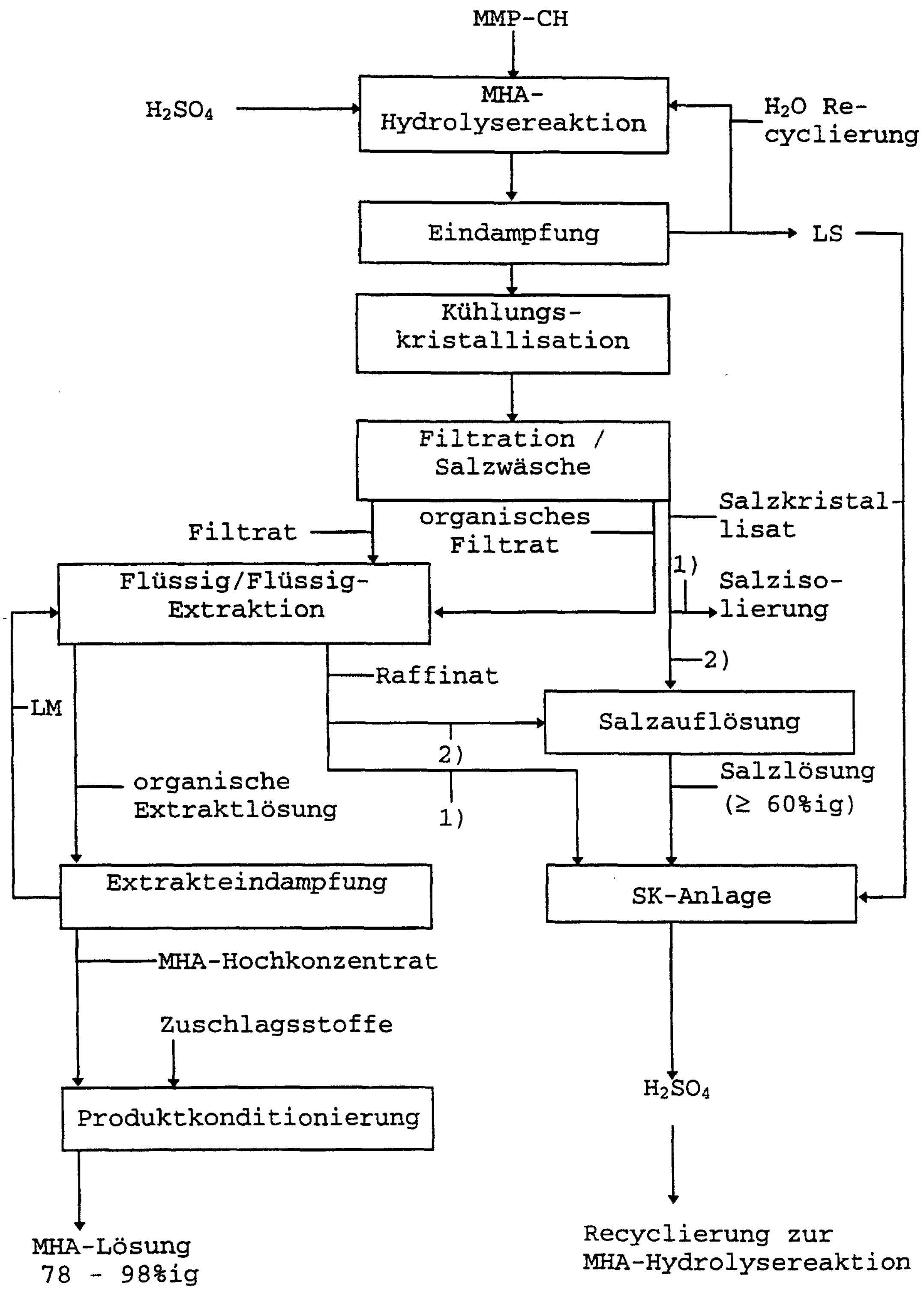

Fig. 3: MHA-Isolierung ohne Salzabtrennung

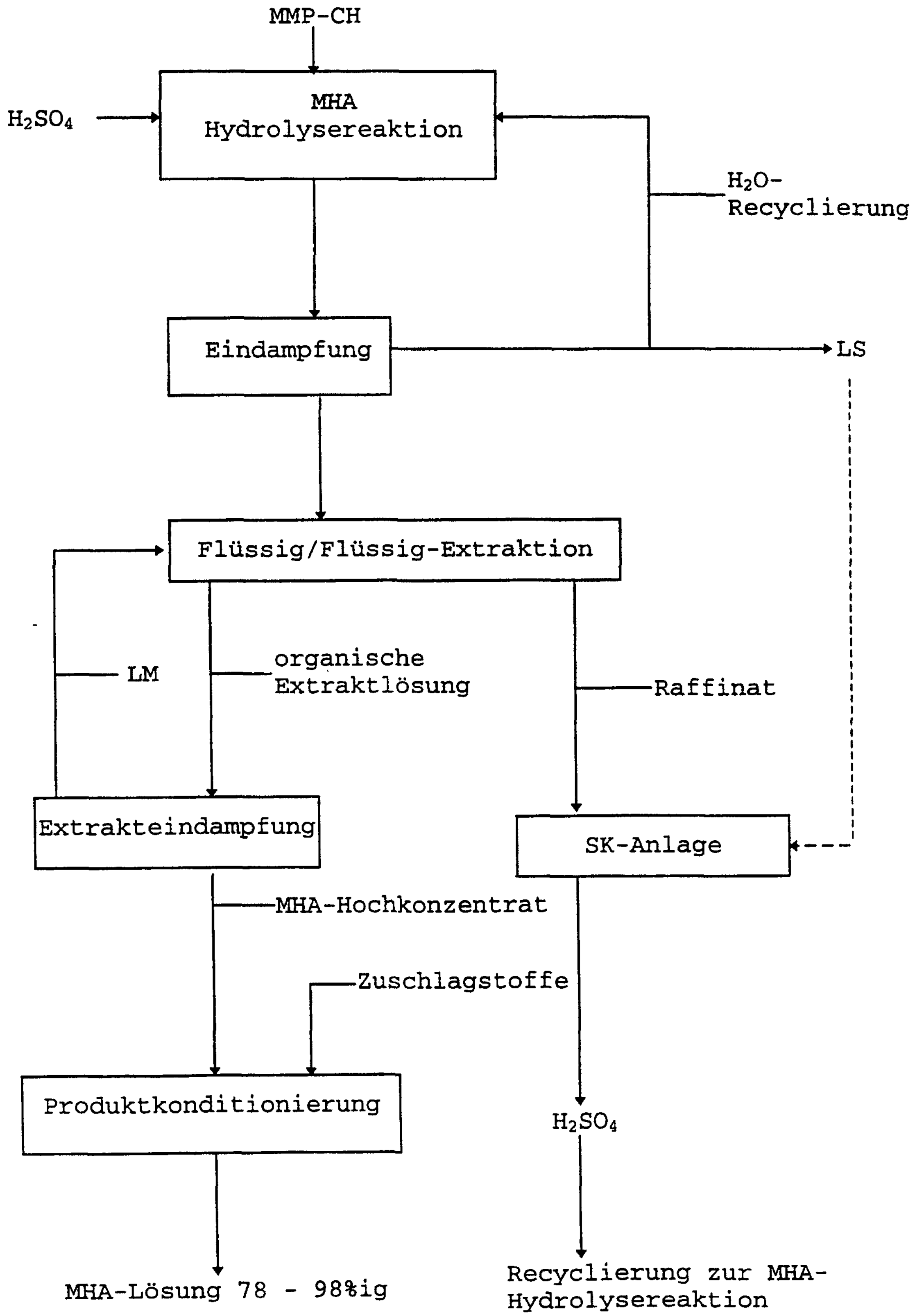

Fig. 4: MHA-Isolierung nach Aufstockung des Salzgehaltes

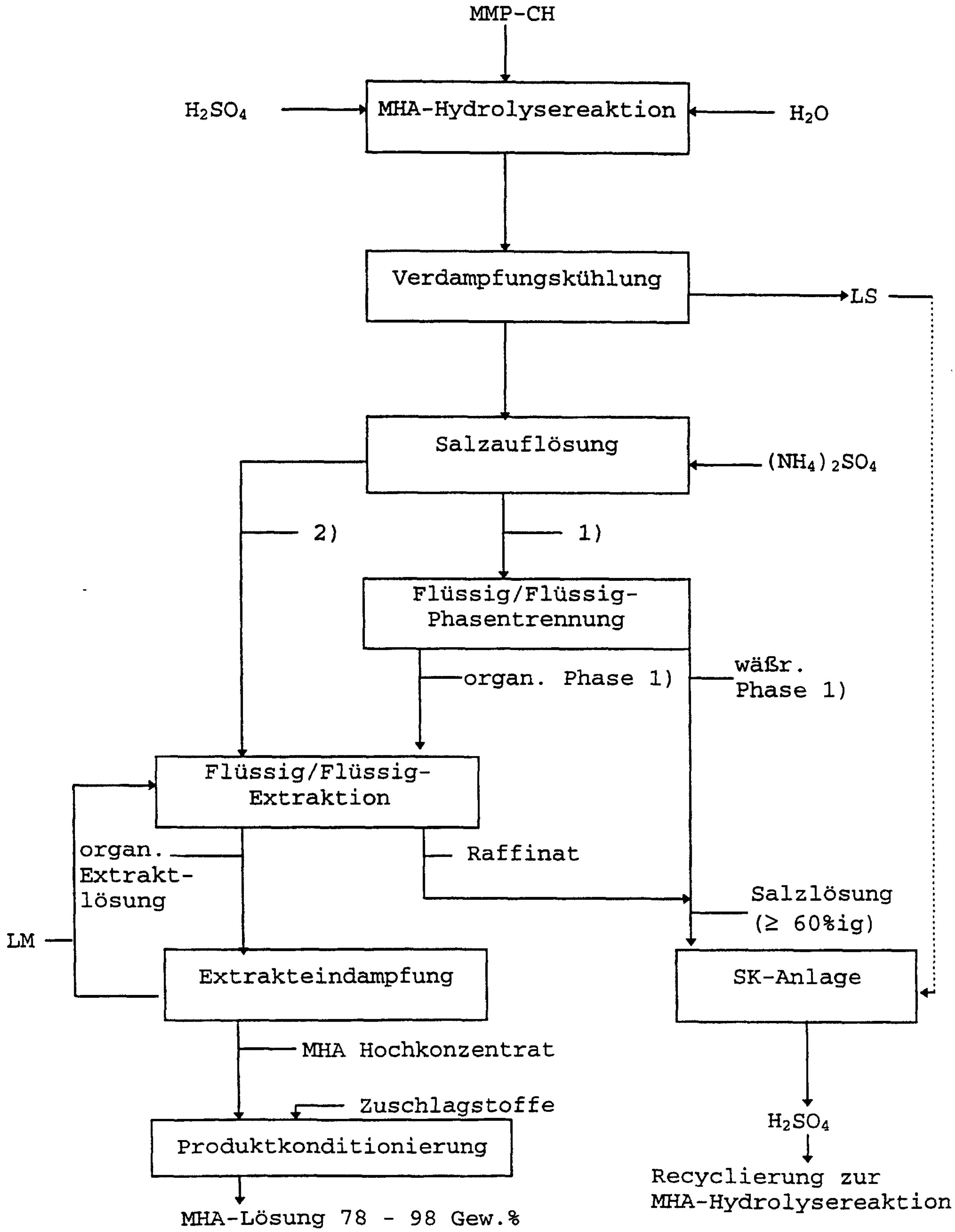

Fig. 5

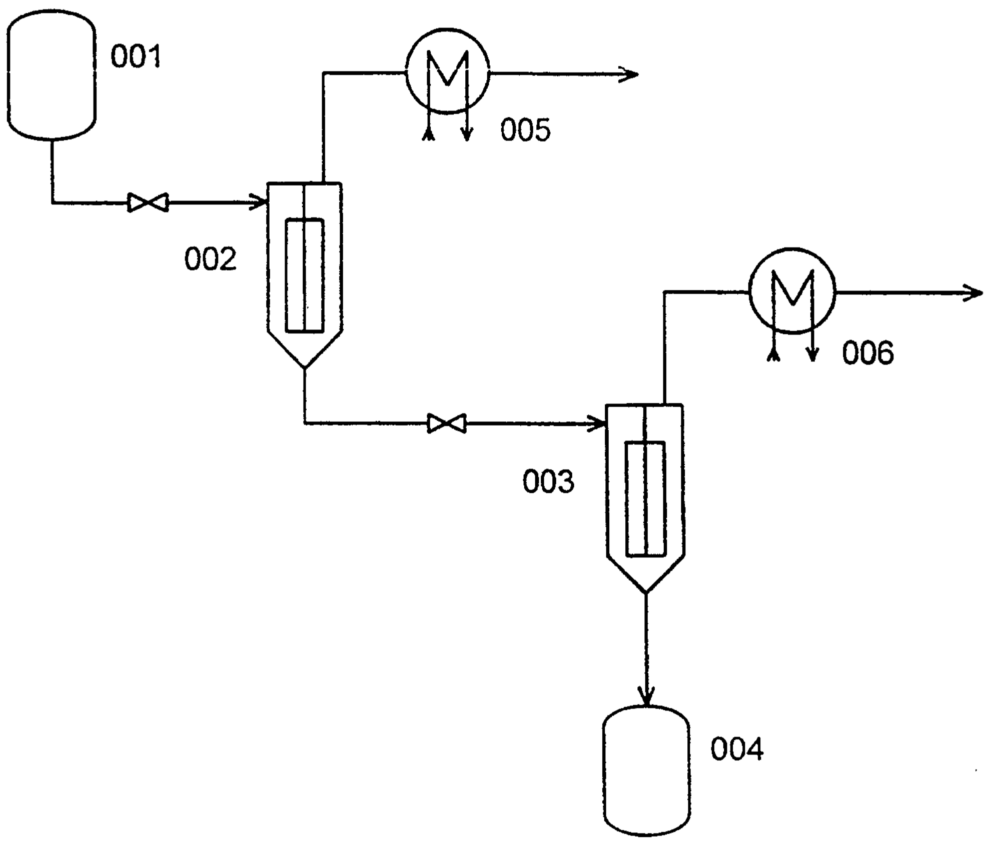

Fig. 6

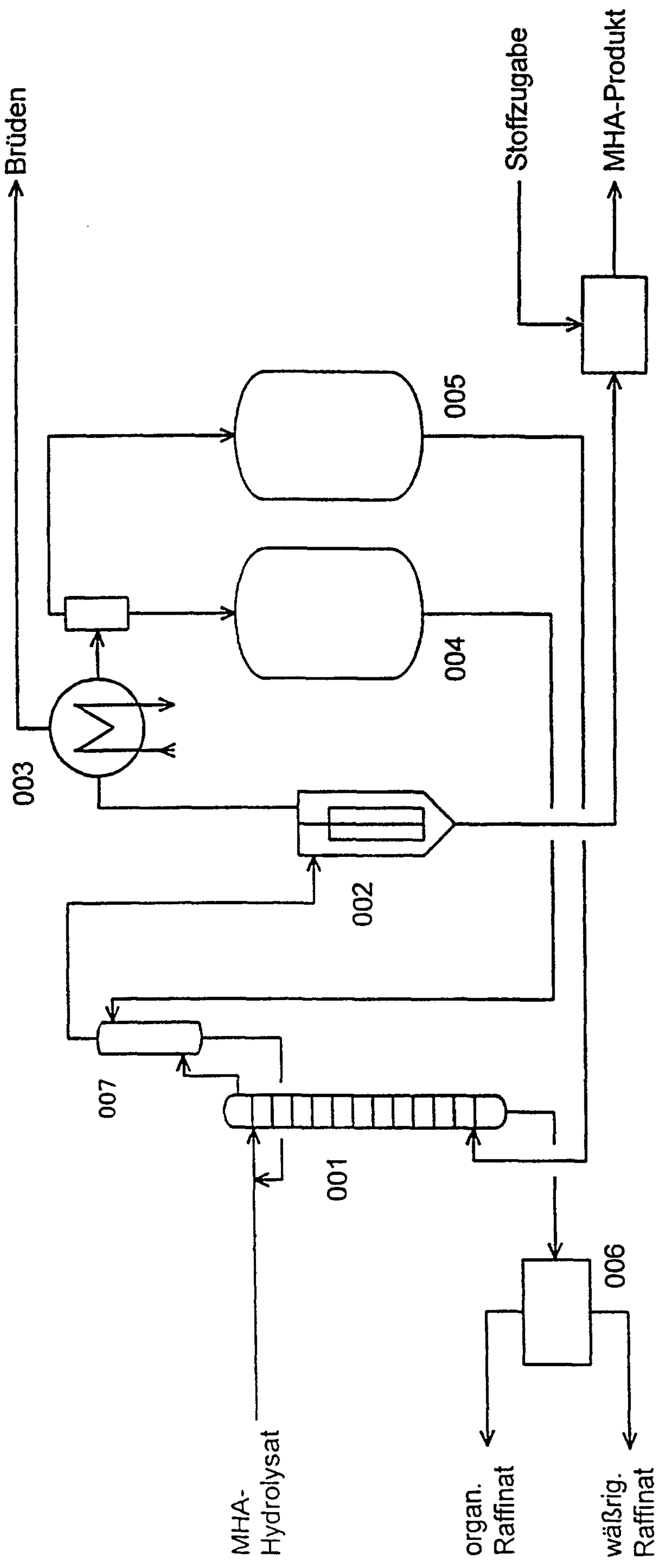
Brüden
Stoffzugabe
MHA-Produkt
003
005
004
002
007
001
006
MHA-Hydrolysat
organ. Raffinat
wäßrig. Raffinat